# FASCICULE DE BREVET EUROPEEN

(11) **EP 1 050 032 B1**
(45) Date de publication et mention de la délivrance du brevet: **24.07.2002**
(21) Numéro de dépôt: 99900922.8
(22) Date de dépôt: 14.01.1999
(51) Int. Cl.: G08B 21/00

(54) **PROCEDE ET DISPOSITIF POUR DETECTER ET PREVENIR L'ENDORMISSEMENT DU CONDUCTEUR D'UN VEHICULE AUTOMOBILE**
VERFAHREN UND VORRICHTUNG ZUR ERFASSUNG UND VERHINDERUNG DES EINSCHLAFENS EINES FAHRZEUGFÜHRERS
METHOD AND DEVICE FOR DETECTING DROWSINESS AND PREVENTING A DRIVER OF A MOTOR VEHICLE FROM FALLING ASLEEP

(30) Priorité: 15.01.1998 FR 9800378
(43) Date de publication de la demande: 08.11.2000
(73) Titulaire: Holding B.E.V. S.A., 2953 Luxembourg (LU)
(72) Inventeur: PIRIM, Patrick, F-75013 Paris (FR)
(74) Mandataire: Michelet, Alain
(86) Numéro de dépôt international: FR9900060
(87) Numéro de publication internationale: WO9936894

(56) Documents cités:
- WO-A-97/01246
- DE-A- 19 715 519
- UENO H ET AL: "DEVELOPMENT OF DROWSINESS DETECTION SYSTEM" PROCEEDINGS OF THE VEHICLE NAVIGATION AND INFORMATION SYSTEMS CONFERENCE, YOKOHAMA, AUG. 31 - SEPT. 2, 1994,31 août 1994, pages 15-20, XP000641294 INSTITUTE OF ELECTRICAL AND ELECTRONICS ENGINEERS

## Description

La présente invention a pour objet un procédé et un dispositif pour surveiller en continu l'état de vigilance du conducteur d'un véhicule automobile, afin de détecter et prévenir une tendance éventuelle à l'endormissement de celui-ci.

On sait qu'une proportion non négligeable, sinon importante, des accidents sur route résultent de l'endormissement, total ou partiel (somnolence), du conducteur d'un véhicule automobile (auto particulière, auto utilitaire, camionnette, camion), avec pour résultat de nombreux morts et blessés.

On voit donc l'intérêt, humain et économique, à éviter l'endormissement d'un conducteur en provoquant une alarme, notamment sonore, dès que celui-ci tend à s'assoupir, afin de l'éveiller.

Pour détecter la tendance à l'endormissement d'un conducteur, on a proposé sur un véhicule automobile
- d'une part, de détecter la variation de l'actionnement du volant par un conducteur qui tend à s'endormir et
- d'autre part, de détecter la variation des déplacements verticaux des paupières d'un conducteur qui tend à s'endormir.

La présente invention met en oeuvre une détection du second type (surveillance des déplacements des paupières) et elle est basée sur une constatation physiologique, à savoir la modification de la durée des clignements des yeux, ainsi qu'éventuellement des intervalles de temps entre deux clignements successifs, donc la cadence des clignements, lorsqu'une personne passe de l'état éveillé à l'état de somnolence précédant l'endormissement de celui-ci : la durée des clignements d'oeil d'une personne est de l'ordre de 100 à 200 ms (millisecondes) lorsqu'elle est éveillée et de l'ordre de 500 à 800 ms lorsqu'elle somnole, tandis que l'intervalle de temps séparant deux clignements successifs, qui est sensiblement constant à l'état éveillé, varie dans une plage relativement large à l'état somnolent. C'est la variation de la durée des clignements qui est essentiellement mise en oeuvre dans le cadre de l'invention.

Le procédé et le dispositif selon l'invention décèlent l'augmentation de la durée des clignements des yeux du conducteur et déclenchent une alarme, sonore ou autre, lorsque cette durée dépasse un seuil déterminé, compris en particulier entre 200 et 500 ms, par exemple égal à 350 ms, ce seuil étant éventuellement modifiable en fonction de la physiologie du conducteur.

Dans la demande de brevet français N° 96.09420 déposée le 26 juillet 1996 et la demande de brevet international (P.C.T.) PCT/FR97 /01354 déposée le 22 juillet 1997, en invoquant la priorité de ladite demande de brevet français, l'inventeur de ces deux demandes étant également l'inventeur de la présente invention, on a décrit un procédé et un dispositif, fonctionnant en temps réel, pour le repérage et la localisation d'une zone en mouvement relatif dans une scène, ainsi que pour la détermination de la vitesse et de la direction du déplacement.

Parmi les applications envisagées de ce procédé et ce dispositif, on a décrit, dans lesdites demandes de brevet, la mise en oeuvre de ceux-ci pour l'observation et la surveillance d'une zone constituée par la tête d'un conducteur automobile, afin de détecter et prévenir l'endormissement de celui-ci.

Selon cette application particulière des procédé et dispositif desdites demandes de brevet :
- on produisait un signal vidéo représentatif, en temps réel, des images successives des yeux du conducteur ;
- on traitait ce signal vidéo pour, successivement et en continu,
   - détecter, dans l'image des yeux de ce conducteur, les déplacements verticaux des paupières représentatifs du clignement de celles-ci ;
   - déterminer la cadence de ces déplacements verticaux et
   - repérer les cadences inférieures à un certain seuil, qui correspond sensiblement à la cadence de clignement à l'état éveillé du conducteur ; et
- on déclenchait une alarme en cas de franchissement de ce seuil vers le bas par lesdites cadences, afin d'éveiller le conducteur.

La présente invention a pour objet des perfectionnements aux procédé et dispositif des demandes de brevet précitées, en ce qui concerne leur application à la surveillance d'un conducteur automobile, afin de détecter sa tendance éventuelle à l'endormissement.

Ainsi l'article de Hiroshi Ueno et al. intitulé « Development of drowsiness detection system », publié dans la revue de l'Institute of Electrical and Electronics Engineers (IEEE) du 31 août 1994 pages 15-20, analyse les différentes techniques de détection de l'assoupissement du conducteur d'un véhicule automobile au volant. En particulier on mentionne la mise en oeuvre d'une caméra vidéo associée à un calculateur de traitement de l'image vidéo, au moyen d'un logiciel réalisant, d'une part, la détection du visage du conducteur, notamment d'un rectangle englobant un oeil, et, d'autre part, du rapport des durées des yeux ouverts et des yeux fermés pour déterminer des critères d'endormissement.

La demande de brevet allemand publiée DE 197 15519 et la demande de brevet français publiée correspondante n° 2.747.346 décrivent un appareil et un procédé d'estimation du niveau de somnolence du conducteur d'un véhicule mettant en oeuvre une caméra vidéo disposée aux pieds du conducteur du véhicule et un calculateur de traitement de l'image fournie par la caméra, avec un logiciel qui détecte les clignements des yeux par détermination du temps écoulé entre le début et la fin des clignements. En particulier, une unité 10 du calculateur réalise
- la mémorisation de l'image vidéo et le traitement de celle-ci afin de déterminer une région comprenant les yeux du conducteur,
- la détection de l'intervalle de temps entre la fermeture des paupières du conducteur et l'ouverture complète de celles-ci; et
- un traitement dans une mémoire 11 et une unité de calcul 22, en combinaison avec l'unité 10, afin de calculer un rapport d'apparition de clignements lents.

La demande de brevet internationale publiée WO 97/01246 a pour objet un système de sécurité comportant une caméra vidéo disposée dans le rétroviseur intérieur d'un véhicule automobile et un écran vidéo déporté pour l'analyse de ce qui se passe dans le véhicule et autour de celui-ci, ainsi que de ce qui s'est passé grâce à l'enregistrement du signal vidéo de sortie de la caméra. Il s'agit en fait d'une caméra cachée (dans le rétroviseur), afin d'échapper aux regards des intrus, et qui observe une aire étendue couvrant l'intérieur et l'environnement du véhicule, l'enregistrement permettant de repérer plus tard ce qui s'est passé dans cette aire (page 6, lignes 13 à 19), et non pas d'un capteur dont l'angle de vision est sensiblement limité au visage du conducteur afin de détecter son endormissement éventuel et provoquer son éveil.

Ces documents antérieurs mettent en oeuvre une véritable caméra vidéo et un calculateur externe, la détection du clignement des yeux dans ledit article et lesdites demandes française et allemande requérant une unité complexe de traitement, tandis que ladite demande internationale ne résout pas le problème de la détection de l'endormissement, le conducteur s'il sommeille n'étant capable de regarder ni l'écran vidéo, ni l'enregistrement des images vidéo. On voit donc que ce document ne concerne pas le même domaine technique que les deux documents précédents.

Par rapport à ces documents antérieurs, la présente invention réalise une meilleure approche de la prise de mesure du clignement des yeux du conducteur ; en effet
- elle permet de mettre en oeuvre dans le rétroviseur du véhicule un simple capteur (éventuellement le capteur d'une caméra vidéo miniature), notamment un capteur MOS, qui n'est pas forcément au format vidéo classique ;
- elle réalise un mouvement de l'axe optique du capteur lié au déplacement du rétroviseur par le conducteur afin de diriger ledit axe vers le visage du conducteur ;
- elle permet une perception visuelle intégrée, un circuit intégré assurant une détection immédiate des mouvements des paupières ;
- elle met en oeuvre un processeur très simple pour le traitement des informations fournies par le capteur ;
- elle permet l'intégration possible dans une puce électronique (chip) du capteur, de l'électronique associée à celui-ci et du calculateur, le tout disposé dans le rétroviseur ;
- elle donne la possibilité d'associer une telle puce disposée à l'intérieur du rétroviseur au processeur prévu dans le tableau de bord, le rétroviseur constituant ainsi un appareil intelligent de détection de l'endormissement, d'un coût raisonnable du fait qu'il comporte, par rapport à un rétroviseur intérieur classique, simplement une puce et un dispositif mécanique d'orientation de cette puce supplémentaire.

Dans ces conditions, l'invention a tout d'abord pour objet un procédé pour surveiller en continu l'état de vigilance du conducteur d'un véhicule automobile, afin de détecter et prévenir une tendance éventuelle à l'endormissement de celui-ci, qui consiste
- à produire un signal vidéo représentatif, en temps réel, des images successives d'au moins le visage du conducteur ;
- à traiter ce signal, successivement et en continu, pour
   - détecter, dans ce signal, la portion correspondant effectivement à l'image de la tête du conducteur,
   - déterminer la valeur d'un paramètre relatif au clignement des paupières, qui se modifie notablement lors du passage de l'état éveillé à l'état somnolent du conducteur de part et d'autre d'un seuil, et
   - repérer, en temps réel, le franchissement, par la valeur de ce paramètre, de ce seuil représentatif du passage de l'état éveillé à l'état somnolent du conducteur ; et
- à déclencher, en réponse au franchissement de ce seuil, une alarme apte à réveiller le conducteur ;
et qui est caractérisé en ce que
- d'une part, le signal vidéo est produit en utilisant un capteur optoélectronique solidaire d'un rétroviseur du véhicule automobile, dimensionné et disposé pour recevoir essentiellement l'image du visage du conducteur en place sur son siège et ayant son axe optique de réception des rayons lumineux dirigé vers la tête du conducteur lorsque le rétroviseur est correctement orienté ; et
- d'autre part, le traitement dudit signal vidéo consiste, après avoir détecté la présence du conducteur à sa place, à, successivement et en continu,
   - détecter, à partir d'une analyse des pixels en déplacement entre deux trames successives de même nature dudit signal vidéo, les déplacements horizontaux du conducteur, afin de cadrer le visage de celui-ci dans les trames correspondantes successives du signal vidéo,
   - détecter, à partir d'une analyse des pixels en déplacement entre deux trames successives de même nature dudit signal vidéo, les déplacements verticaux dans le visage, ainsi cadré, du conducteur, afin de cadrer les yeux de celui-ci,
   - déterminer, à partir d'une analyse des pixels en déplacement entre deux trames successives de même nature dudit signal vidéo, les durées successives des clignements des yeux, ainsi cadrés, de celui-ci, ces durées constituant le dit paramètre,
   - comparer ces durées successives des clignements, ainsi déterminées, à un seuil représentatif du passage de l'état éveillé à l'état somnolent du conducteur, et
   - déclencher, lorsque les durées de clignement dépassent vers le haut le dit seuil, une alarme apte réveiller le conducteur.

Avantageusement ledit capteur est placé dans le boîtier du rétroviseur derrière la glace de celui-ci qui est constituée par un miroir sans tain, l'axe optique de réception dudit capteur étant symétrique à un axe orienté dans le plan vertical médian dudit véhicule, par rapport à un axe orthogonal au dit miroir sans tain.

De préférence, on détecte la présence du conducteur à sa place en déterminant le nombre de pixels correspondants dans les trames successives de même nature du signal vidéo pour lesquels un déplacement significatif est détecté et en comparant ce nombre au nombre total de pixels par trame du signal vidéo, afin de déterminer si le rapport entre le nombre de pixels représentant un déplacement et le nombre total de pixels par trame dépasse un seuil représentatif du passage de l'absence de conducteur à sa place à la présence d'un conducteur à sa place.

Le procédé peut, dans des modes de réalisation préférés, comprendre en outre une ou plusieurs des caractéristiques suivantes :
- entre les phases de détection des déplacements horizontaux, afin de cadrer le visage du conducteur, et de détection des déplacements verticaux, afin de cadrer les yeux de celui-ci, on prévoit une phase de cadrage large des yeux en se limitant à une portion du visage cadré englobant les yeux et leur environnement immédiat, par application du rapport anthropométrique entre ladite portion et le visage entier d'une personne ;
- simultanément à la phase de détermination des durées de clignement des yeux, on prévoit une phase de détermination des intervalles de temps séparant deux clignements successifs de ceux-ci et on déclenche une alarme renforcée dès que ces intervalles de temps présentent une irrégularité qui dépasse un seuil déterminé ;
- on réactualise en continu les données concernant au moins un des paramètres suivants :
   déplacements horizontaux, déplacements verticaux, durées des clignements des yeux, intervalles entre clignements successifs, afin de perfectionner les approximations des valeurs normales de ces paramètres pour le conducteur effectivement présent et à l'état éveillé ;
- les différentes phases successives du procédé sont réalisées au moyen de programmes informatiques successifs portant sur le traitement des valeurs successives des pixels correspondants des trames de même nature du signal vidéo obtenu à partir dudit capteur.

La présente invention a également pour objet un dispositif pour surveiller en continu l'état de vigilance du conducteur d'un véhicule automobile, afin de détecter et prévenir une tendance éventuelle à l'endormissement de celui-ci, qui met en oeuvre le procédé susvisé et qui est caractérisé en ce qu'il comprend, en combinaison :
a) un capteur optoélectronique, qui, en combinaison avec une électronique associée, élabore, en réponse à la réception de rayons lumineux, un signal vidéo à trames de même nature, ou correspondantes, successives, ledit capteur étant solidaire d'un rétroviseur du véhicule automobile et dimensionné et disposé pour recevoir essentiellement l'image du visage du conducteur en place sur son siège et ayant son axe optique de réception des rayons lumineux dirigé vers la tête du conducteur lorsque le rétroviseur est correctement orienté; et
b) au moins un circuit intégré comportant
   - des moyens pour détecter la présence du conducteur à sa place dans le véhicule, et pour élaborer un signal de présence ;
   - des moyens, activés par ce signal de présence, pour détecter, à partir d'une analyse des pixels en déplacement entre deux trames successives de même nature dudit signal vidéo, les déplacements horizontaux de dit conducteur, afin de cadrer le visage de celui-ci dans les trames successives de même nature dudit signal vidéo, et pour élaborer un signal de fin de cadrage de visage ;
   - des moyens, activés par ledit signal de fin de cadrage du visage, pour détecter, à partir d'une analyse des pixels en déplacement entre deux trames successives de même nature de la portion des trames successives de même nature dudit signal vidéo correspondant au cadrage du visage, les déplacements verticaux dans le visage, ainsi cadré, du conducteur, afin de cadrer les yeux de celui-ci dans ladite portion des trames de ce signal, et pour élaborer un signal de fin de cadrage des yeux du conducteur ;
   - des moyens, activés par ledit signal de fin de cadrage des yeux, pour déterminer, à partir d'une analyse des pixels en déplacement entre deux trames successives de même nature de la portion des trames successives de même nature dudit signal vidéo correspondant au cadrage des yeux, les durées successives des clignements des yeux du conducteur ;
   - des moyens pour comparer ces durées successives des clignements, ainsi déterminées, à un seuil représentatif du passage de l'état éveillé à l'état somnolent du conducteur ; et
   - des moyens pour déclencher, lorsque les durées des clignements dépassent ledit seuil, une alarme apte à réveiller le conducteur.

Avantageusement, dans ledit dispositif, ledit capteur est placé dans le boîtier du rétroviseur derrière le miroir de celui-ci, qui est un miroir sans tain, ledit capteur étant porté par une première extrémité d'une première tige traversant, à travers une rotule, un étrier porté par le boitier du rétroviseur, à l'intérieur de celui-ci, la seconde extrémité de cette tige étant articulée librement, au moyen d'un joint, à la première extrémité d'une seconde tige traversant, à travers une rotule, le boitier du rétroviseur, tandis que la seconde extrémité de ladite seconde tige est fixée à la carrosserie du véhicule au dessus du pare-brise, de manière que l'axe optique de réception du dit capteur soit symétrique à un axe orienté dans le plan vertical médian dudit véhicule, par rapport à un axe orthogonal au dit miroir sans tain.

De préférence, lesdits moyens pour détecter la présence du conducteur à sa place et pour élaborer un signal de présence sont constitués par des moyens pour déterminer le nombre de pixels dans les trames successives de même nature dudit signal vidéo pour lesquels un déplacement significatif est détecté, des moyens pour comparer ledit nombre au nombre total de pixels par trame du signal vidéo, afin de déterminer si le rapport entre le nombre de pixels correspondant à un déplacement et le nombre total de pixels par trame dépasse un seuil représentatif du passage de l'état d'absence de conducteur à sa place à l'état de présence d'un conducteur à sa place.

Le dispositif peut, dans des modes de réalisation préférés, comprendre en outre un ou plusieurs des moyens suivants, à savoir :
- des moyens, activés par ledit signal de fin de cadrage du visage, pour sélectionner, dans ladite portion des trames successives dudit signal vidéo correspondant au cadrage du visage, une portion réduite correspondant à un cadrage large, ou grossier, des yeux du conducteur englobant les yeux et leur environnement immédiat par application du rapport anthropométrique entre ledit cadrage large et le visage entier d'une personne et des moyens pour élaborer un signal de fin de cadrage large des yeux, ce signal activant lesdits moyens pour détecter les déplacements verticaux dans le visage du conducteur ;
- des moyens, fonctionnant en parallèle avec lesdits moyens pour déterminer les durées successives des clignements des yeux et donc activés par ledit signal de fin de cadrage des yeux, pour déterminer les intervalles de temps séparant deux clignements successifs et pour déclencher une alarme renforcée dès que ces intervalles de temps présentent une irrégularité qui dépasse un seuil déterminé ;
- des moyens pour réactualiser en continu les données concernant au moins un des paramètres suivants : déplacements horizontaux, déplacements verticaux, durées des clignements des yeux, intervalles entre clignements successifs, afin de perfectionner les approximations des valeurs normales du paramètre impliqué pour le conducteur effectivement présent et à l'état éveillé.

Avantageusement l'ensemble capteur - unité électronique de traitement est réalisé comme décrit et illustré dans les deux demandes de brevet susmentionnées ayant le même inventeur que la présente.

L'invention a également pour objet, à titre de produit industriel nouveau, un rétroviseur de véhicule automobile, caractérisé en ce que son miroir est constitué par une glace sans tain et en ce qu'il comporte, derrière cette glace, un capteur optoélectronique qui coopère avec une unité électronique telle que décrite dans la demande de brevet français N° 96.09420 déposée le 26 juillet 1996 et la demande de brevet international (P.C.T.) PCT/FR97/01354 déposée le 22 juillet 1997, cette unité étant également disposée à l'intérieur du rétroviseur et étant apte à déclencher un dispositif d'alarme dès que ladite unité détermine que les mouvements verticaux des paupières d'une personne regardant la face avant de ladite glace correspondent à une durée des clignements des yeux qui dépasse un seuil prédéterminé inclus dans l'intervalle temporel compris entre la durée des clignements d'une personne éveillée et celle d'une personne qui somnole.

De préférence ledit rétroviseur porte au moins une diode électroluminescente au moins dans l'infra-rouge qui est activée au moins lorsque la luminosité ambiante devient insuffisante pour éclairer le visage du conducteur, ledit capteur optoélectronique étant sensible, entre autres, aux radiations infra-rouges émises par ladite diode.

Avantageusement, dans le dispositif ainsi que le rétroviseur selon l'invention, le capteur, ladite électronique associée et ledit circuit intégré sont constituées par une puce électronique (chip) disposée à l'intérieur du boitier du rétroviseur.

On va décrire maintenant un mode de réalisation préféré d'un dispositif selon l'invention, mettant en oeuvre le procédé selon l'invention, ainsi que certaines variantes de celui-ci, avec référence aux dessins annexés, sur lesquels :
Les figures 1 et 2 sont des vues, respectivement de coté et par-dessus, illustrant schématiquement la tête d'un conducteur de véhicule automobile et ses axes de vision vers l'avant et vers l'arrière.
La figure 3 illustre schématiquement la disposition classique du miroir d'un rétroviseur intérieur dans un véhicule automobile et les différents axes de vision du conducteur, cette figure correspondant à l'état de la technique.
Les figures 4 et 5 représentent respectivement l'ensemble et les articulations d'un rétroviseur avec le capteur optoélectronique et son électronique associée dans le cadre de l'invention.
La figure 6 illustre le champ du capteur optoélectronique prévu dans le rétroviseur des figures 4 et 5.
Les figures 7 et 8 représentent la manière de cadrer le visage du conducteur en place.
Les figures 9 et 10 représentent la manière de cadrer les yeux du conducteur en place.
Les figures 11 et 12 sont relatives à la mesure de la durée des clignements des yeux du conducteur et des intervalles temporels séparent deux clignements successifs.
La figure 13 représente l'ordinogramme des phases successives de fonctionnement.
La figure 14 illustre les avantages de la mise en oeuvre, comme capteur, d'un capteur de type MOS.
La figure 15, enfin, est une variante de la figure 9, la zone d'observation privilégiée englobant le nez, en plus des yeux.

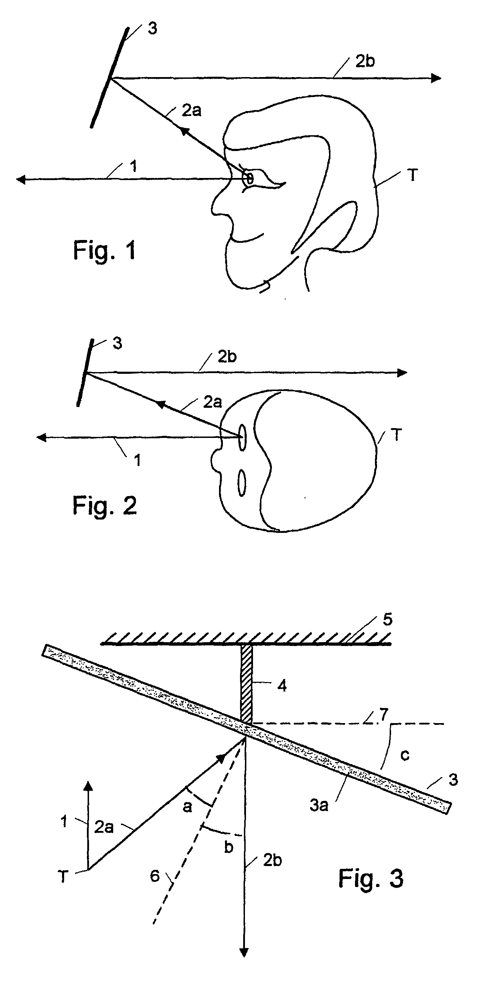

En se référant tout d'abord aux figures 1 à 6, on va commencer la description détaillée du mode de réalisation préféré de l'invention par celle du dispositif optique et mécanique avec le capteur optoélectronique (micro-caméra vidéo ou capteur MOS avec lentille incorporée) et son ensemble électronique associé, constitué essentiellement par une ou plusieurs puces, qui transforme l'image captée par le capteur en un signal vidéo qui est traité afin de détecter une tendance à l'endormissement du conducteur en place, observé par ledit capteur.

En effet l'invention utilise essentiellement la variation de la durée des clignements des yeux d'une personne lors du passage de l'état éveillé à l'état somnolent ou assoupi de celle-ci : une personne éveillée cligne, à intervalles relativement réguliers, des paupières, et donc des cils, en 100 à 200 ms environ, tandis que la durée des clignements de cette personne à l'état somnolent passe à 500 à 800 ms environ, les intervalles entre clignements augmentant et étant variables.

Dans le signal vidéo en provenance du capteur optoélectronique à 50 ou 60 trames correspondantes (de même nature) par seconde, on réalise une détection toutes les 20 ms ou 16,66 ms respectivement, ce qui permet de distinguer facilement des durées de 100 à 200 ms ou de 500 à 800 ms (5 à 10 trames pour l'état éveillé ou au contraire 25 à 40 trames pour l'état somnolent dans le cas de 50 trames de même nature par seconde) et donc de distinguer l'état éveillé de l'état somnolent ou assoupi d'une personne.

Pour une utilisation d'une telle distinction dans le cas du conducteur d'un véhicule automobile, il est désirable de visualiser au mieux la face du conducteur, c'est-à-dire de diriger l'axe optique d'entrée ou réception dudit capteur vers le visage de celui-ci. Le moyen prévu dans le mode de réalisation préféré de l'invention consiste à profiter du fait qu'un conducteur dirige le rétroviseur de son véhicule vers son visage de manière qu'il ait une vue vers l'arrière du véhicule par réflexion sur le miroir du rétroviseur.

On rappelle, avec référence aux figures 1 à 3, le fonctionnement des rétroviseurs classiques logés à l'intérieur d'un véhicule en position centrale, en étant fixés, avec possibilité d'ajustement de l'orientation de leur miroir, sur une portion de la carrosserie à l'intérieur du véhicule.

Les figures schématiques 1 et 2 montrent, vue de coté et de dessus respectivement, la tête *T* d'un conducteur qui peut observer la rue ou route sur laquelle se trouve son véhicule, d'une part, devant lui (flèche 1) et, d'autre part, derrière lui (flèches 2*a* et 2*b)* grâce au miroir 3 du rétroviseur convenablement orienté. Lesdites flèches 1, 2*a*, 2*b* représentent le parcours des rayons lumineux, 2*b* correspondant au rayon réfléchi sur le miroir 3.

En considérant maintenant la figure schématique 3, qui représente le miroir 3 du rétroviseur, miroir fixé par un bras 4 sur une portion 5 de la carrosserie à l'intérieur du véhicule, avec possibilité d'orientation, on retrouve les axes de visée ou flèches 1, 2*a*, 2*b* des figures 1 et 2. On peut noter que les axes ou flèches 1 et 2*b* sont parallèles et sont dirigés suivant la direction de la rue ou de la route.

Sur cette figure 3, on a également représenté, mais en traits interrompus, l'axe optique 6 perpendiculaire à la face 3*a* du miroir 3 d'un rétroviseur intérieur qui divise l'angle formé par les directions 2*a* et 2*b* en deux moitiés égales (angles *a* et *b* égaux) d'après les lois de la réflexion, et l'axe 7 perpendiculaire à l'axe 2*b* et donc parallèle à la portion de support 5, l'angle *c* entre les directions 7 et 3*a* étant égal aux angles *b* et *a*.

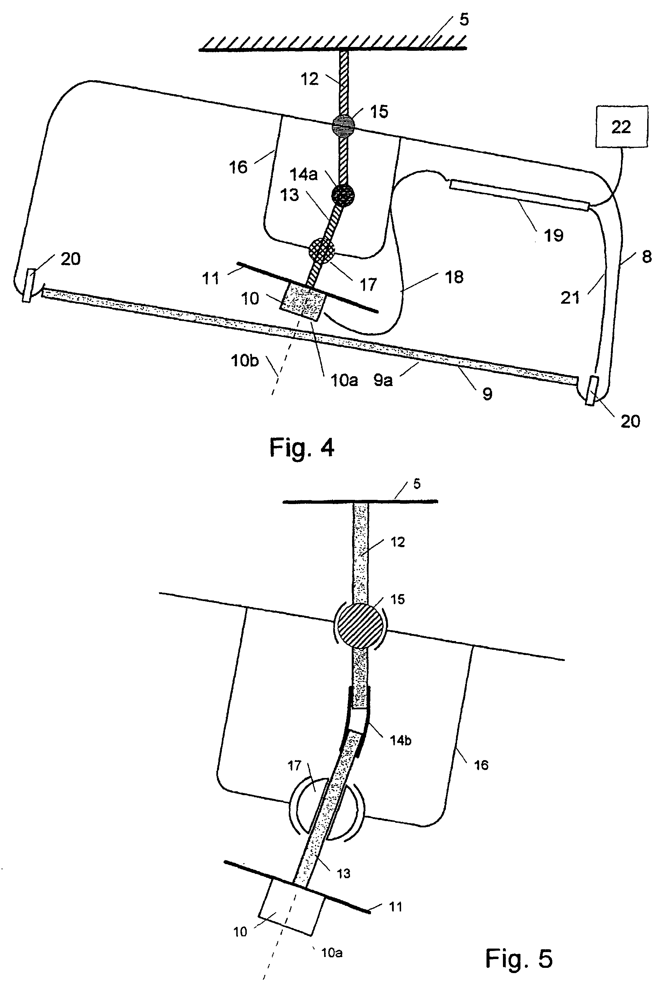

Ces principes de fonctionnement des rétroviseurs intérieurs étant rappelés, on va maintenant avec référence aux figures 4 et 5, exposer le montage mécanique permettant de diriger effectivement l'axe optique d'entrée du capteur optoélectronique vers le visage du conducteur en place, en profitant du fait que le miroir 3 d'un rétroviseur est orienté par le conducteur en place, lorsque cela n'est pas déjà le cas, pour que l'axe 2*a* de visée par le conducteur soit dirigé vers la tête *T* de celui-ci. En effet, si l'axe optique d'entrée du capteur est effectivement dirigé vers la face de conducteur, le signal vidéo produit par celui-ci contiendra les informations nécessaires pour déterminer la durée des clignements des yeux de celui-ci.

Tout d'abord dans le cadre du mode de réalisation préféré de l'invention, le rétroviseur 8 comprend, contrairement au rétroviseurs classiques, une glace sans tain 9 (figure 4) dont la face 9*a* dirigée vers le conducteur joue le même rôle que la face 3*a* du miroir 3 d'un rétroviseur classique (figure 3), mais qui permet à un capteur 10 (constitué par une micro-caméra électronique ou un capteur MOS à lentille incorporée), porté par un support 11 (tournant avec le miroir sans tain 9), de recevoir au moins l'image du visage du conducteur en place lorsque le miroir sans tain 9 (avec le rétroviseur 8) est convenablement orienté par le conducteur pour percevoir la rue ou la route derrière lui ou est déjà ainsi orienté (comme c'est le cas pour le miroir classique 3 de la figure 3).

L'articulation mécanique type Cardan, illustrée sur les figures 4 et 5 (cette dernière figure étant une vue plus détaillée d'une portion de la figure 4), permet l'orientation automatique correcte du support 11, avec le capteur 10, par le conducteur lorsqu'il règle son rétroviseur ou lorsque celui-ci est déjà réglé, et donc de la face réceptrice 10*a* du capteur 10 pour qu'elle reçoive l'image du visage du conducteur en place, son axe optique d'entrée 10*b* étant dirigé vers la tête du conducteur en place du fait de l'angle entre le miroir 9 et le support 11 du capteur 10.

A cet effet l'articulation pour le support 11 comprend deux tiges 12 et 13 articulées librement entre elles par une rotule 14*a* (figure 4) ou un manchon 14*b* (figure 5.). La tige 12 est fixée à une portion 5 de la carrosserie par une de ses extrémités et traverse le boîtier du rétroviseur 8 grâce à la rotule 15 (constituée par une bille et deux calottes sensiblement hémisphériques) avant de pénétrer par son autre extrémité dans le manchon 14*b* ou être fixée à la rotule 14*a*, tandis que la tige 13 porte rigidement, à une extrémité, le support 11 du capteur 10 et traverse l'étrier 16 du rétroviseur 8 grâce à une rotule creuse 17 (à bille traversée par un canal dans lequel est engagée la tige 13 et tournant dans deux calottes sensiblement hémisphériques portées par l'étrier 16) avant de rejoindre par son autre extrémité la rotule 14a ou le manchon 14*b*.

Une telle articulation, qui maintient en permanence un angle approprié entre le miroir 9 et le support 11, permet à la fois l'orientation habituelle du rétroviseur intérieur par le conducteur et l'orientation du support 11 du capteur 10 pour que la face 10*a* de ce capteur reçoive l'image d'au moins le visage du conducteur en place lorsque le rétroviseur est convenablement orienté.

Le capteur optoélectronique 10 débite par un conducteur 18 dans une unité électronique d'analyse 19 (avantageusement constituée par un boîtier à puce ou puces logé à l'intérieur du rétroviseur 8) le signal vidéo qu'il élabore à partir de l'image qu'il reçoit sur sa face 10*a*.

On peut prévoir des diodes électroluminescentes 20 pour émettre, en direction du conducteur en place, lorsque le rétroviseur est correctement orienté, un rayonnement infrarouge apte à éclairer au moins le visage de conducteur en place, lorsque la lumière d'ambiance (y compris celle du tableau de bord) est insuffisante pour le fonctionnement correct du capteur 10, qui dans ce cas doit être sensible au rayonnement infrarouge, et de son unité électronique 19 ; l'excitation, éventuellement progressive, de ces diodes est, par exemple, contrôlée par l'unité électronique 19 grâce à une cellule photoélectrique (non représentée) ou en réponse à des signaux de pixels (dans le signal vidéo) d'intensité insuffisante (comme représenté schématiquement par le conducteur 21).

L'alarme activée, en cas d'endormissement du conducteur, par l'unité électronique 19 est illustrée schématiquement en 22 sur le figure 4, sur laquelle on n'a pas illustré les alimentations du capteur 10, de l'unité électronique 19 et des diodes 20, pour simplifier cette figure.

L'unité 19 pourrait, en variante, être disposée hors du boîtier du rétroviseur.

On va maintenant exposer le traitement, dans l'unité électronique d'analyse 19, du signal vidéo issu du capteur optoélectronique 10 (à micro-caméra électronique ou capteur MOS avec lentille incorporée suivie d'une unité électronique), ce signal vidéo comportant une succession de trames correspondantes (de même nature) à la cadence de 50 ou 60 telles trames par seconde (soit les trames paires ou bien impaires dans le cas d'un signal à deux trames entrelacées par image, soit les trames uniques dans le cas d'un signal à une seule trame par image) ; ce traitement a pour objet de réaliser la surveillance de la vigilance du conducteur en place en déterminant, en temps réel et en continu, la durée des clignements de ses yeux et en déclenchant, en cas de tendance du conducteur à l'endormissement (révélée par la variation de cette durée), un signal d'alarme apte à éveiller celui-ci.

Le procédé et le dispositif, selon la présente invention mettant en oeuvre, pour repérer et localiser une zone en mouvement (à savoir successivement le conducteur, son visage et ses yeux, en particulier ses paupières) et déterminer la direction et éventuellement la vitesse de ce mouvement, le procédé et le dispositif selon les demandes de brevet susvisées, dont les descriptions sont incorporées dans la présente description détaillée par référence, il est utile de résumer le processus décrit dans ces demandes de brevet.

Dans ces demandes, le signal vidéo (produit par une caméra vidéo ou autre capteur), qui comprend une succession de trames de même nature (constituées par les trames correspondantes, soit paires, soit impaires, dans le cas d'un système vidéo à deux trames entrelacées par image, soit les trames successives dans le cas d'un système vidéo à trame unique par image), est traité pour successivement
- déduire, des variations de la valeur ou intensité de chaque pixel entre une trame et la trame correspondante antérieure,
   - d'une part, un signal binaire, noté *DP,* dont les deux valeurs possibles sont représentatives, l'une, d'une variation significative de la valeur du pixel et, l'autre, d'une non-variation significative de cette valeur, valeurs notées par exemple «1» et «0» respectivement, et
   - d'autre part, un signal numérique, noté *CO,* à nombre réduit de valeurs possibles, ce signal étant représentatif de la grandeur de cette variation de la valeur du pixel ;
- répartir suivant une matrice, par roulement, des valeurs de ces deux signaux *DP* et *CO* pour une même trame qui défile à travers la matrice ; et
- déduire, de cette répartition matricielle, le déplacement recherché et ses paramètres (localisation, direction et vitesse).

Cette dernière opération de détection du déplacement met en préférence en oeuvre, selon ces demandes de brevet précitées,
- la formation d'histogrammes, suivant deux axes, par exemple O*x* et O*y* orthogonaux, d'au moins les signaux *DP* et *CO,* répartis matriciellement dans l'opération précédente, et
- le repérage, dans chacun des histogrammes relatifs à *DP* et *CO,* d'un domaine de variation significative de *CO* avec simultanément *DP* = «1».

La présente invention, réalise successivement, par mise en oeuvre du procédé et dispositif selon les demandes de brevet précitées, dont on vient de résumer le processus,
- dans une phase préliminaire, la détection de la présence d'un conducteur en place ;
- dans une première phase, le cadrage du visage du conducteur dans les trames de même nature, ou correspondantes, successives du signal vidéo ;
- dans une deuxième phase, le cadrage des yeux du conducteur à l'intérieur du cadrage du visage ;
- dans une troisième phase, la détermination des durées successives des clignements des yeux du conducteur, et éventuellement la détermination des intervalles de temps séparant deux clignements successifs ;
- dans une quatrième phase, la comparaison des durées des clignements à un certain seuil, avec génération d'un signal d'alarme apte à éveiller le conducteur dès que cette comparaison révèle le dépassement vers le haut de ce seuil par cette durée, et éventuellement la comparaison des variations temporelle des intervalles de temps entre deux clignements successifs à un autre seuil, avec génération d'un signal d'alarme renforcé dès que cette comparaison révèle le dépassement vers le haut de ce dernier seuil.

On va décrire maintenant plus en détail la réalisation de chacune de ces cinq phases par le procédé et le dispositif selon l'invention.

La phase préliminaire, qui détecte la présence d'un conducteur en place et amorce la première phase de cadrage du visage, est déclenchée par un contacteur actionné manuellement ou autrement, notamment par mise en oeuvre des procédé et dispositif des demandes de brevet précitées ; elle commence effectivement avec le réglage du rétroviseur pour orienter la face avant 9*a* du miroir sans tain 9 de celui-ci (figure 4) vers le conducteur afin qu'il aperçoive dans ce miroir la rue ou route derrière lui, au cas il y a besoin d'un tel réglage.

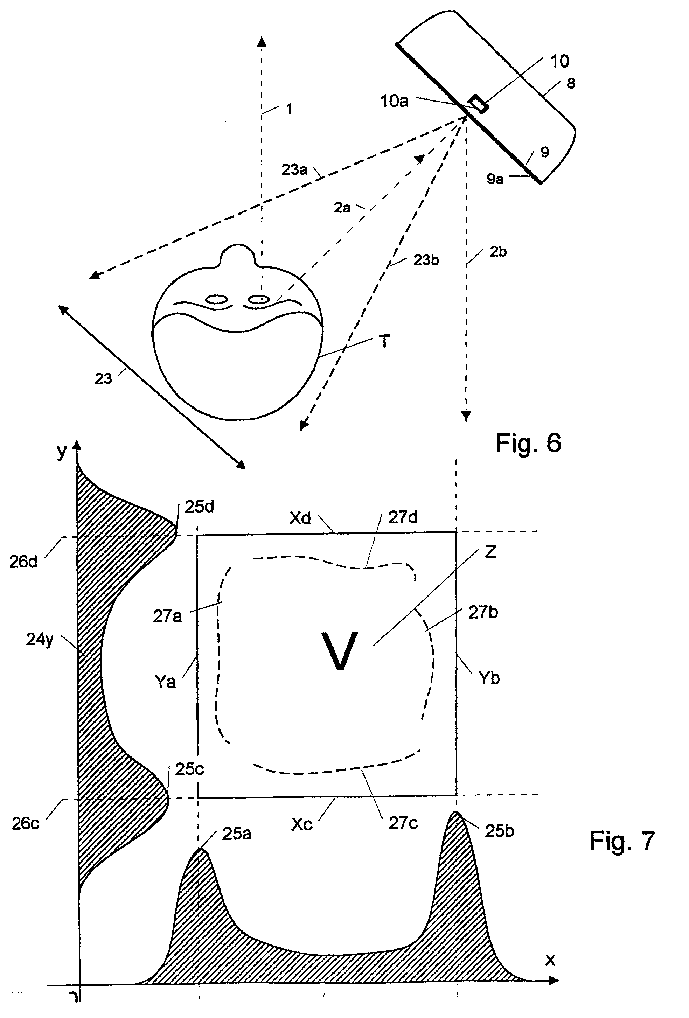

La figure 6 illustre, entre les directions 23*a* et 23*b*, le champ 23 du capteur 10, la tête *T* du conducteur devant se trouver, du fait du réglage du rétroviseur intérieur 8, tel que décrit avec référence aux figures 4 et 5, à l'intérieur et dans la zone centrale de ce champ conique 23. Ce champ peut être relativement étroit, étant donné que les déplacements de la tête *T* du conducteur au cours de la conduite sont limités (sauf rares exceptions) ; la limitation du champ améliore la sensibilité du dispositif étant donné que l'image du visage du conducteur, qui est reçue par la face 10*a* du capteur correctement orienté en même temps que le miroir 9, occupe alors une place relativement importante dans les trames du signal vidéo ; elle est donc représentée par un nombre de pixels qui est une fraction notable du nombre total des pixels par trame.

Sur la figure 6 on retrouve les directions ou rayons lumineux 1, 2*a* et 2*b* de la figure 3.

La mise en place du conducteur est avantageusement détectée par les déplacements de sa tête, en particulier de son visage, pour venir en position de conduite, par mise en oeuvre du procédé et du dispositif selon les deux demandes de brevet précitées qui permettent de détecter les déplacements, comme rappelé brièvement ci-dessus.

En fait l'arrivée du conducteur à sa place et le déplacement de sa tête *T* en résultant sont révélés par le nombre important de pixels du signal vidéo pour lesquels le signal binaire *DP* a la valeur «1» correspondant à une variation significative de la valeur du pixel entre deux trames correspondantes successives et le signal numérique *CO* a une valeur relativement élevée.

Le rapport du nombre de tels pixels (avec *DP* et *CO* ayant les valeurs définies ci-dessus) au nombre total de pixels d'une trame, lors de la mise en place du conducteur, dépend de la dimension du champ de vision du capteur de part et d'autre de la tête *T* en place pour la conduite. En cas de champ de vision étroit (angle réduit entre 23*a* et 23*b* figure 6), on peut considérer par exemple, que si plus de la moitié des pixels «en déplacement» d'une trame ont un *DP* et un *CO* avec les valeurs sus-avancées, il y a mise en place du conducteur. On peut alors considérer un seuil de 50 % entre le nombre de pixels «en déplacement» et le nombre total de pixels d'une trame et dans ce cas la phase préliminaire se termine par la production, lorsque ce seuil est dépassé vers le haut, d'un drapeau «1» de présence qui amorce la suite du traitement du signal vidéo, en commençant par la première phase. Bien entendu le seuil retenu pour le déclenchement du drapeau «1» peut être différent de 50 %, en tenant compte du champ de vision du capteur 10.

En variante, le drapeau «1» de présence amorçant la première phase peut être produit par une commande externe à l'unité électronique 19, mais déclenchant celle-ci, par exemple provoquée par l'actionnement de la clé de contact, le bouclage de la ceinture de sécurité du conducteur ou le fléchissement du siège du conducteur sous son poids.

Lorsque la présence du conducteur a été révélée et le drapeau «1» de présence généré, la première phase de traitement du signal vidéo peut commencer. Elle consiste, comme indiqué précédemment, à cadrer le visage du conducteur dans le signal vidéo en éliminant les portions superflues, en haut, en bas, à droite et à gauche de la tête dans l'image perçue par le détecteur 10.

A cet effet, par mise en oeuvre du procédé et du dispositif selon l'invention, ce sont les déplacements horizontaux, c'est-à-dire de la droite vers la gauche et inversement, qui sont détectés, car la tête d'un conducteur a tendance à se déplacer horizontalement plutôt que verticalement, c'est-à-dire de haut en bas et inversement.

On extrait, donc, du flot des données représentées dans les trames correspondantes successives du signal vidéo, un signal de déplacement horizontal, en position, sens et éventuellement vitesse, grâce à la matrice roulante des valeurs de *DP* et *CO,* et on l'analyse par sélection suivant deux axes de coordonnées privilégiés, par exemple les axes classiques O*x* et O*y* des coordonnées cartésiennes, par mise en oeuvre des moyens de formation d'histogrammes des demandes de brevet précitées.

La comptabilisation, en fin de trames, des pixels représentatifs d'un déplacement horizontal permet de détecter des pics de déplacement le long des bords du visage, pour lesquels les variations de luminosité, donc de valeur de pixel, sont les plus importantes, aussi bien en projection horizontale suivant O*x* qu'en projection verticale suivant O*y* par exemple.

Ceci est illustré sur la figure 7 sur laquelle on a représenté les axes *Ox* et *Oy,* ainsi que les histogrammes 24*x*, suivant *Ox,* et 24*y*, suivant *Oy,* c'est-à-dire en projection horizontale et verticale respectivement.

Les pics 25*a* et 25*b*, de l'histogramme 24*x*, et 25*c* et 25*d,* de l'histogramme 24*y*, délimitent, par leur coordonnés respectives 26*a*, 26*b*, 26*c*, 26*d,* un cadre limité par les droites *Ya*, *Yb*, *Xc, Xd* qui renferme le visage *V* du conducteur entouré par les ondulations respectives 27*a*, 27*b,* 27*c*, 27*d* qui illustrent les légers mouvements du conducteur dans les zones de plus grande variation des intensités des pixels, lors de ses mouvements.

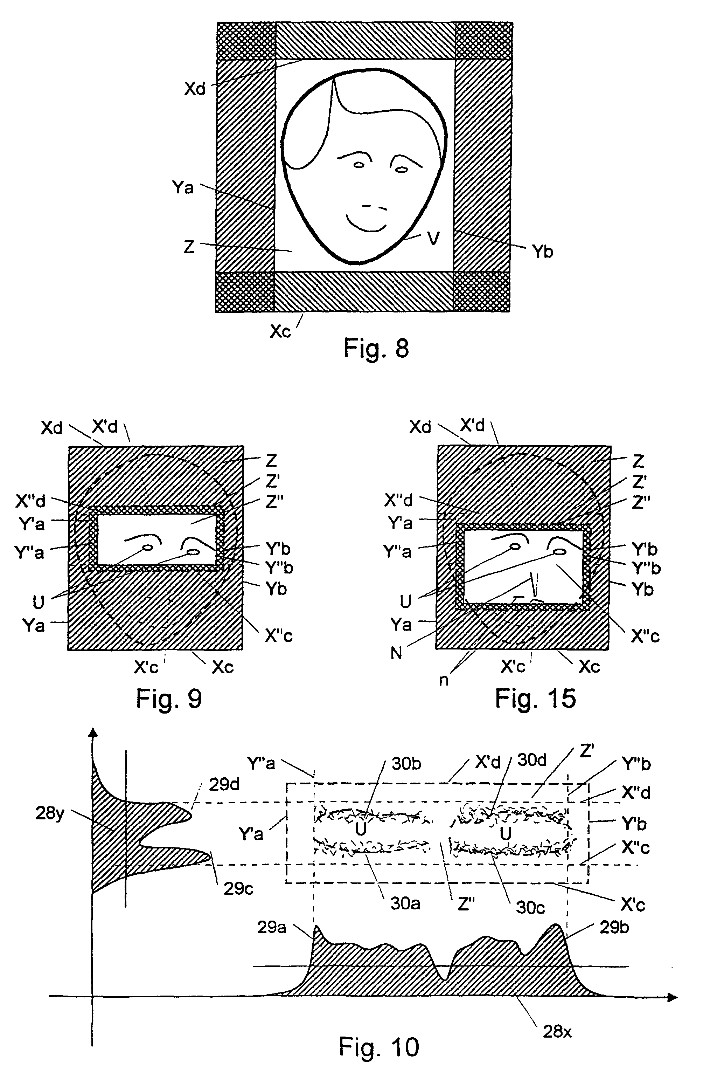

Le repérage des coordonnées 26*a*, 26*b,* 26*c* et 26*d,* correspondant aux quatre pics 25*a*, 25*b*, 25*c* et 25*d* des deux histogrammes 24*x* et 24*y*, permet donc de mieux définir et cadrer l'emplacement du visage *V* du conducteur dans la zone Z et d'éliminer, pour la suite du traitement du signal vidéo, les portions supérieure, inférieure, de droite et de gauche par rapport au cadre *Xc, Xd, Ya, Yb,* comme illustré sur la figure 8 par des zones hachurées encadrant le visage *V*, ce qui permet d'accroître la précision, et éventuellement la cadence, de l'analyse portant sur la zone centrale *Z,* non hachurée, encadrée par les droites *Xc, Xd, Ya*, *Yb* et contenant le visage *V.*

Cette opération de cadrage du visage entier est renouvelée à intervalles réguliers, par exemple toutes les dix trames du signal vidéo, et les valeurs moyennes (au cours du temps) des coordonnées 26*a*, 26*b*, 26*c*, 26*d*, sont déterminées, en redéfinissant le cadre, légèrement variable, mais relativement stable, *Xc, Xd*, *Ya*, *Yb* autour du visage *V*. On constate donc que la position dudit cadre (avec la zone limitée pour l'analyse ultérieure) est très robuste, c'est-à-dire stable au cours du temps.

Un nouveau drapeau «1» de visage cadré est produit après établissement du cadrage du visage *V* du conducteur.

La production de ce drapeau déclenche la deuxième phase, qui consiste à réduire encore plus le cadre du traitement, à savoir à celui des yeux du conducteur.

Cette deuxième phase comporte, de préférence, une opération préliminaire consistant à utiliser, dans l'unité électronique 19, le rapport anthropométrique habituel entre la zone des yeux et l'ensemble du visage chez un être humain, notamment dans le sens vertical, la zone des yeux occupant seulement une portion limitée du visage entier.

L'unité électronique 19 détermine alors, dans cette opération préliminaire, par ratio un cadre *Z'* plus limité, incluant les yeux *U* du conducteur, dans le cadre précédent *Z* du visage *V,* limité par *Ya*, *Yb*, *Xc, Xd*, ce cadre *Z'* étant, comme illustré sur la figure 9 défini par les droites *Y'a*, *Y'b*, *X'c* et *X'd* à l'intérieur du cadre *Ya*, *Yb, Xc, Xd* (zone *Z*).

On élimine ainsi les zones hachurées externes (simples hachures) sur la figure 9 pour ne conserver que le cadre *Z'*, ce qui facilite le cadrage définitif des yeux dans la deuxième phase et augmente sa précision et la vitesse de sa détermination.

Après la fin de cette opération préliminaire si elle existe, ce qui génère un drapeau «1» de cadrage grossier des yeux, ou directement après la première phase de traitement, c'est-à-dire respectivement en réponse à l'apparition du drapeau «1» de cadrage grossier des yeux ou du drapeau «1» de visage cadré respectivement, l'unité électronique 19 effectue la deuxième phase de cadrage effectif plus serré des yeux du conducteur en détectant, dans la matrice des *DP* et *CO*, les emplacements de pixels pour lesquels *DP* = *1* et *CO* présente une valeur élevée, notamment pour des déplacements dans le sens vertical du fait que les paupières clignent de haut en bas et inversement.

Lorsque le nombre de tels emplacements de pixels atteint un certain seuil dans le cadre *Y'a*, *Y'b*, *X'c*, *X'd* (zone *Z'*) dans le cas où l'opération préliminaire est prévue ou dans le cadre *Ya*, *Yb*, *Xc, Xd* (zone *Z*) en l'absence d'une telle opération préliminaire, ce seuil étant par exemple de 20 % par rapport au nombre total de pixels dans le cadre *Y'a*, *Y'b*, *X'c, X'd* dans le premier cas et de 10 % par rapport au nombre total de pixels dans le cadre *Ya, Yb, Xc, Xd* dans le second cas, un drapeau «1» de cadrage fin des yeux est généré ; ce drapeau indique en fait que les paupières du conducteur sont actives, car il est provoqué par les clignements des yeux du conducteur ; mouvements dans le sens vertical repérés de la même manière que les déplacements horizontaux du visage du conducteur dans la première phase.

Sur la figure 10 on a illustré le cadre éventuel *Y'a, Y'b, X'c, X'd,* définissent la zone *Z'* de cadrage grossier des yeux du conducteur, ainsi que les histogrammes 28*x* selon l'axe *Ox* et 28*y* suivant l'axe *Oy* des déplacements verticaux des paupières du conducteur, c'est-à-dire des pixels de la matrice révélant, par leur *DP* et leur *CO,* de tels déplacements. Ces histogrammes 28*x* et 28*y*, qui correspondent aux histogrammes 24*x* et 24*y* des déplacements horizontaux du visage du conducteur, illustrés sur la figure 7, déterminent, par leurs pics 29*a*, 29*b,* 29*c,* 29*d,* des droites horizontales *X"c* et *X"d* et des droites verticales *Y"a* et *Y"b* définissant, à l'intérieur de la zone *Z'*, une zone *Z"* qui encadre les yeux du conducteur dont les déplacements des bords sont indiqués en 30*a* et 30*b* pour un oeil et 30*c* et 30*d* pour l'autre oeil.

La position du cadre *Y"a*, *Y"b*, *X"c*, *X"d* est réactualisée par détermination des valeurs moyennes au cours du temps, par exemple toutes les dix trames, des coordonnées des pics 29*a*, 29*b,* 29*c,* 29*d* et, à partir de la production du drapeau «1» de cadrage fin des yeux, ce sont seulement les pixels compris dans le cadre limité de la zone *Z"* qui sont traités dans la troisième phase déclenchée par ce drapeau (la zone *Z"* étant figurée en blanc sur la figure 9).

Dans cette troisième phase sont déterminées les durées des clignements des yeux, et éventuellement les intervalles de temps séparant deux clignements successifs, en perfectionnant l'analyse des déplacements verticaux des paupières dans la zone *Z"* par traitement dans l'unité électronique 19 des portions des trames successives du signal vidéo correspondant à cette zone *Z"*, ce qui permet une grande précision.

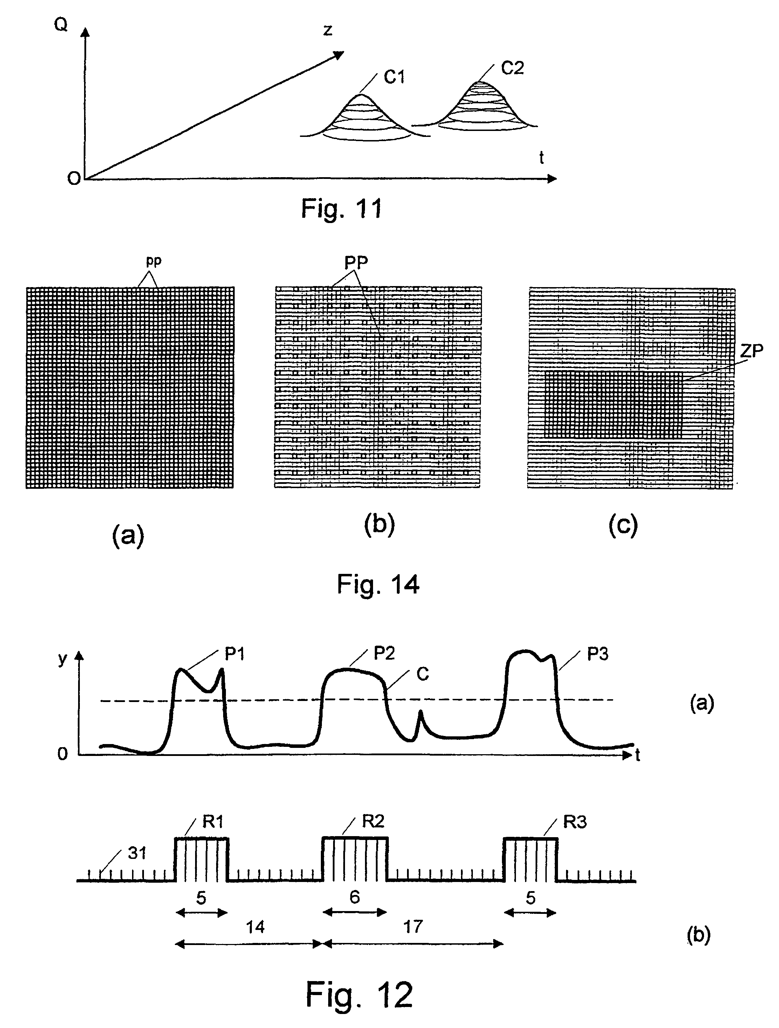

Sur la figure 11 on a illustré dans un système de coordonnées suivant trois directions orthogonales entre elles, à savoir *OQ* sur laquelle on a porté *CO,* c'est-à-dire les intensités de la variation de la valeur de pixel, correspondant au mouvement vertical des paupières, *Ot* sur laquelle on a porté les intervalles de temps entre deux clignements successifs et *Oz* sur laquelle on a porté les durées des clignements, donc trois paramètres différents permettant de déterminer le passage de l'état éveillé à l'état endormi du conducteur. Deux clignements successifs *C*_{*1*} et *C*_{*2*} sont représentés sur la figure 11.

La figure 12 illustre par la courbe *C,* sur la portion (*a*), la variation, dans le temps suivant *Ot* du nombre de pixels par trame en mouvement vertical significatif (pour lesquels *DP* = *1* et *CO* a une valeur relativement importante), les pics successifs *P*_{*1*}, *P*_{*2*}, *P*_{*3*} du nombre de pixels en mouvement correspondant à des clignements.

Les trames correspondantes successives relatives à la courbe *C* sont représentées, schématiquement et en partie, sur la portion (*b*) de la figure 12, par des traits verticaux, tels que 31, dont les pics *P*_{*1*}, *P*_{*2*}, *P*_{*3*} sont encadrés par des rectangles *R*_{*1*}*, R*_{*2*}, *R*_{*3*} respectivement, les deux portions (*a*) et *(b)* de la figure 12 étant disposées, l'une sous l'autre, en synchronisme temporel. Sur cette figure 12 on a représenté enfin les durées des clignements (5,6,5) et les intervalles de temps (14, 17) entre clignements successifs, en nombre de trames, valeurs qui correspondent à l'état éveillé du conducteur.

L'unité électronique 19, dans cette troisième phase, calcule les durées successives des clignements des yeux et les intervalles de temps successifs entre deux clignements consécutifs et fait une analyse statistique bi-dimensionnelle entre les durées successives des clignements et les intervalles entre clignements. Elle établit si les durées des clignements dépassent un certain seuil, par exemple 350 ms, et dans ce cas déclenchent un drapeau «1» de seuil de clignement dépassé et éventuellement si les intervalles de temps entre deux clignements successifs sont relativement constants ou au contraire significativement variables dans le temps, et dans le second cas déclenchent un drapeau «1» d'intervalles entre clignements variables.

Le premier drapeau sert à déclencher une alarme, sonore par exemple, apte à réveiller le conducteur, tandis que le second drapeau renforce l'alarme, par exemple en augmentant le niveau sonore.

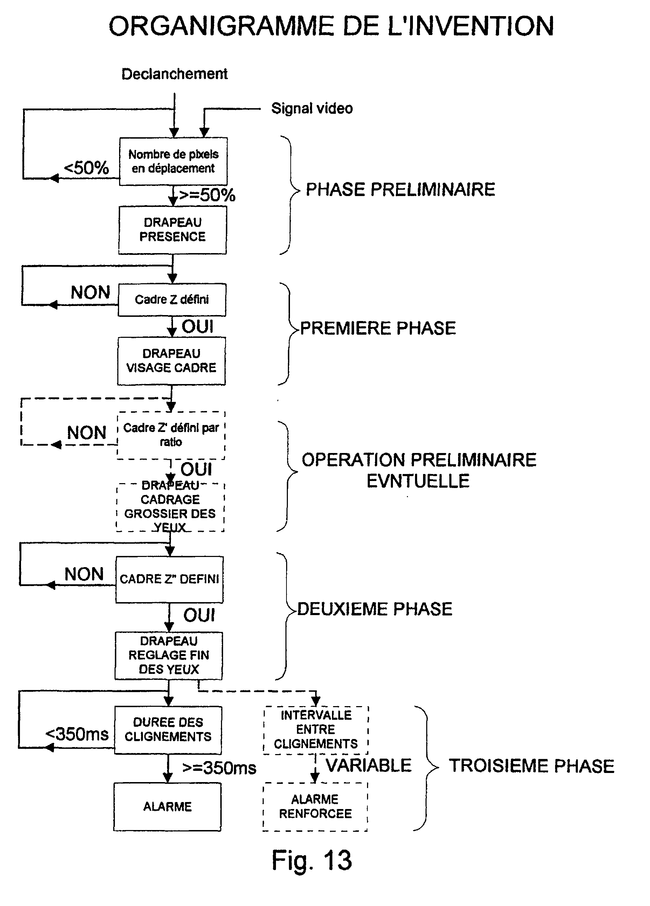

L'ordinogramme annexé à titre de planche 6 (figure 13) résume les différentes phases successives.

Le dialogue avec l'extérieur est réalisé, de préférence en mode série (CAN - VAN).

L'ensemble du capteur, de l'électronique associée et du processeur ou calculateur peut-être avantageusement intégré dans une puce électronique (chip) logée dans le rétroviseur interne du véhicule.

Le rétroviseur des figures 4 et 5 convient aussi bien pour un conducteur occupant le siège gauche que le siège droit, pour les pays à conduite à droite, et peut éventuellement être un rétroviseur extérieur, notamment du côté du conducteur.

Il est particulièrement intéressant d'utiliser un capteur de type MOS, qui permet une détermination de la valeur de pixel, position par position de pixel, sans être obligé, comme dans le cas d'un capteur CCD d'extraire les valeurs de pixels ligne par lignes et position par position dans chaque ligne.

Comme représenté sur la figure 14, il est alors possible de réaliser une sélection variable des positions de pixels : au lieu (a) d'examiner toutes les positions pp de pixels sur la totalité de l'image (grand nombre de traitements), on peut (b) examiner seulement certains pixels maillés PP formant un réseau régulier représentant la globalité de l'image du visage du conducteur et son environnement immédiat (effet de zoom) ; enfin on peut (c) sélectionner une zone particulière ZP, à savoir celle des yeux en traitant seulement les pixels de cette zone.(puissance de traitement constante)

Sur la figure 15, qui est une variante de la figure 9, dont elle reprend les références, on a prévu de sélectionner pour la phase finale de surveillance du conducteur, non seulement la région des yeux (comme le cas de la figure 9), mais également des narines n du nez N, l'observation des déplacements du contour ou taches sombres des narines permettent d'améliorer le maintien de la zone d'observation incluant les yeux.

Comme il va de soi, l'invention n'est pas limitée au mode de réalisation préféré décrit et illustré, ni à ses variantes mentionnées ci-dessus ; l'invention englobe au contraire les modifications, variantes et perfectionnement entrant dans le cadre des définitions de l'invention données dans le préambule et les revendications jointes.

## Revendications

1. Procédé pour surveiller en continu l'état de vigilance du conducteur d'un véhicule automobile, afin de détecter et prévenir une tendance éventuelle à l'endormissement de celui-ci,
qui consiste
- à produire un signal vidéo représentatif, en temps réel, des images successives d'au moins le visage du conducteur ;
- à traiter ce signal, successivement et en continu, pour
• détecter, dans ce signal, la portion correspondant effectivement à l'image de la tête du conducteur,
• déterminer la valeur d'un paramètre relatif au clignement des paupières, qui se modifie notablement lors du passage de l'état éveillé à l'état somnolent du conducteur de part et d'autre d'un seuil, et
• repérer, en temps réel, le franchissement, par la valeur de ce paramètre, de ce seuil représentatif du passage de l'état éveillé à l'état somnolent du conducteur ; et
- à déclencher, en réponse au franchissement de ce seuil, une alarme apte à réveiller le conducteur ;
et qui est **caractérisé en ce que**
- d'une part, le signal vidéo est produit en utilisant un capteur optoélectronique solidaire d'un rétroviseur du véhicule automobile, dimensionné et disposé pour recevoir essentiellement l'image du visage du conducteur en place sur son siège et ayant son axe optique de réception des rayons lumineux dirigé vers la tête du conducteur lorsque le rétroviseur est correctement orienté ; et
- d'autre part, le traitement dudit signal vidéo consiste, après avoir détecté la présence du conducteur à sa place, à, successivement et en continu,
• détecter, à partir d'une analyse des pixels en déplacement entre deux trames successives de même nature dudit signal vidéo, les déplacements horizontaux du conducteur, afin de cadrer le visage de celui-ci dans les trames correspondantes successives du signal vidéo,
• détecter, à partir d'une analyse des pixels en déplacement entre deux trames successives de même nature dudit signal vidéo, les déplacements verticaux dans le visage, ainsi cadré, du conducteur, afin de cadrer les yeux de celui-ci,
• déterminer, à partir d'une analyse des pixels en déplacement entre deux trames successives de même nature dudit signal vidéo, les durées successives des clignements des yeux, ainsi cadrés, de celui-ci, ces durées constituant le dit paramètre,
• comparer ces durées successives des clignements, ainsi déterminées, à un seuil représentatif du passage de l'état éveillé à l'état somnolent du conducteur, et
• déclencher, lorsque les durées de clignement dépassent vers le haut le dit seuil, une alarme apte réveiller le conducteur.

2. Procédé selon la revendication 1, **caractérisé en ce que** ledit capteur est placé dans le boîtier du rétroviseur derrière la glace de celui-ci qui est constituée par un miroir sans tain, l'axe optique de réception (2a) dudit capteur étant symétrique à un axe (2b) orienté dans le plan vertical médian dudit véhicule, par rapport à un axe (6) orthogonal au dit miroir sans tain.

3. Procédé selon la revendication 1 ou 2, **caractérisé en ce qu'**on détecte la présence du conducteur à sa place en déterminant le nombre de pixels correspondants dans les trames successives de même nature du signal vidéo pour lesquels un déplacement significatif est détecté et en comparant ce nombre au nombre total de pixels par trame du signal vidéo, afin de déterminer si le rapport entre le nombre de pixels représentant un déplacement et le nombre total de pixels par trame dépasse un seuil représentatif du passage de l'absence de conducteur à sa place à la présence d'un conducteur à sa place.

4. Procédé selon la revendication 1, 2, ou 3 **caractérise en ce qu'**entre les phases de détection des déplacements horizontaux, afin de cadrer le visage du conducteur, et de détection des déplacements verticaux, afin de cadrer les yeux de celui-ci, on prévoit une phase de cadrage large des yeux en se limitant à une portion du visage cadré englobant les yeux et leur environnement immédiat, par application du rapport anthropométrique entre ladite portion et le visage entier d'une personne.

5. Procédé selon l'une quelconque des revendications précédentes, **caractérisé en ce,** simultanément à la phase de détermination des durées des clignements des yeux, on prévoit une phase de détermination des intervalles de temps séparant deux clignements successifs de ceux-ci et on déclenche une alarme renforcée dès que ces intervalles de temps présentent une irrégularité qui dépasse un seuil déterminé.

6. Procédé selon l'une quelconque des revendications précédentes, **caractérisé en ce qu'**on réactualise en continu les données concernant au moins un des paramètres suivants : déplacements horizontaux, déplacements verticaux, durées des clignements des yeux, intervalles entre clignements successifs, afin de perfectionner les approximations des valeurs normales de ces paramètres pour le conducteur effectivement présent et à l'état éveillé.

7. Procédé selon l'une quelconque des revendications précédentes, **caractérisé en ce que** les différentes phases successives du procédé sont réalisées au moyen de programmes informatiques successifs portant sur le traitement des valeurs successives des pixels correspondants des trames de même nature du signal vidéo obtenu à partir dudit capteur.

8. Dispositif pour surveiller en continu l'état de vigilance du conducteur d'un véhicule automobile, afin de détecter et prévenir une tendance éventuelle à l'endormissement de celui-ci, qui met en oeuvre le procédé selon l'une quelconque des revendications 1 à 7 et qui est **caractérisé en ce qu'**il comprend, en combinaison :
a) un capteur optoélectronique (10), qui, en combinaison avec une électronique associée (19), élabore, en réponse à la réception de rayons lumineux, un signal vidéo à trames de même nature, ou correspondantes, successives, ledit capteur étant solidaire d'un rétroviseur (8) du véhicule automobile et dimensionné et disposé pour recevoir essentiellement l'image du visage du conducteur en place sur son siège et ayant son axe optique (10b) de réception des rayons lumineux dirigé vers la tête (T) du conducteur lorsque le rétroviseur est correctement orienté ; *et*
b) au moins d'un circuit intégré comportant
- des moyens pour détecter la présence du conducteur à sa place dans le véhicule, et pour élaborer un signal de présence ;
- des moyens, activés par ce signal de présence, pour détecter, à partir d'une analyse des pixels en déplacement entre deux trames successives de même nature dudit signal vidéo, les déplacements horizontaux de dit conducteur, afin de cadrer le visage (V) de celui-ci dans les trames successives de même nature dudit signal vidéo, et pour élaborer un signal de fin de cadrage de visage ;
- des moyens, activés par ledit signal de fin de cadrage du visage, pour détecter, à partir d'une analyse des pixels en déplacement entre deux trames successives de même nature de la portion des trames successives de même nature dudit signal vidéo correspondant au cadrage du visage, les déplacements verticaux dans le visage, ainsi cadré, du conducteur, afin de cadrer les yeux (U) de celui-ci dans ladite portion des trames de ce signal, et pour élaborer un signal de fin de cadrage des yeux du conducteur ;
- des moyens, activés par ledit signal de fin de cadrage des yeux, pour déterminer, à partir d'une analyse des pixels en déplacement entre deux trames successives de même nature de la portion des trames successives de même nature dudit signal vidéo correspondant au cadrage des yeux, les durées successives des clignements des yeux du conducteur ;
- des moyens pour comparer ces durées successives des clignements, ainsi déterminées, à un seuil représentatif du passage de l'état éveillé à l'état somnolent du conducteur ; et
- des moyens pour déclencher, lorsque les durées des clignements dépassent ledit seuil, une alarme (22) apte à réveiller le conducteur.

9. Dispositif selon la revendication 8, **caractérisé en ce que** ledit capteur (10) est placé dans le boîtier du rétroviseur (8) derrière le miroir de celui-ci, qui est un miroir (9) sans tain, ledit capteur (10) étant porté par une première extrémité d'une première tige (13) traversant, à travers une rotule (17), un étrier (16) porté par le boitier du rétroviseur (8), à l'intérieur de celui-ci, la seconde extrémité de cette tige (13) étant articulée librement, au moyen d'un joint (14a,14b), à la première extrémité d'une seconde tige (12) traversant, à travers une rotule (15), le boitier du rétroviseur (8), tandis que la seconde extrémité de ladite seconde tige (12) est fixée à la carrosserie du véhicule (en 5) au dessus du pare-brise, de manière que l'axe optique de réception (2a) du dit capteur soit symétrique à un axe (2b) orienté dans le plan vertical médian dudit véhicule, par rapport à un axe orthogonal (6) au dit miroir sans tain.

10. Dispositif selon la revendication 8 ou 9,**caractérisé en ce que** lesdits moyens pour détecter la présence du conducteur à sa place et pour élaborer un signal de présence sont constitués par des moyens pour déterminer le nombre de pixels dans les trames successives de même nature dudit signal vidéo pour lesquels un déplacement significatif est détecté, des moyens pour comparer ledit nombre au nombre total de pixels par trame du signal vidéo, afin de déterminer si le rapport entre le nombre de pixels correspondant à un déplacement et le nombre total de pixels par trame dépasse un seuil représentatif du passage de l'état d'absence de conducteur à sa place à l'état de présence d'un conducteur à sa place.

11. Dispositif selon la revendication 8, 9 ou 10, **caractérisé en ce qu'**il comprend en outre des moyens, activés par ledit signal de fin de cadrage du visage, pour sélectionner, dans ladite portion des trames successives dudit signal vidéo correspondant au cadrage du visage, une portion réduite correspondant à un cadrage large, ou grossier, des yeux du conducteur englobant les yeux et leur environnement immédiat par application du rapport anthropométrique entre ledit cadrage large et le visage entier d'une personne et des moyens pour élaborer un signal de fin de cadrage large des yeux, ce signal activant lesdits moyens pour détecter les déplacements verticaux dans le visage du conducteur.

12. Dispositif selon l'une quelconque des revendications 8 à 11, **caractérisé en ce qu'**il comporte des moyens, fonctionnant en parallèle avec lesdits moyens pour déterminer les durées successives des clignements des yeux et donc activés par ledit signal de fin de cadrage des yeux, pour déterminer les intervalles de temps séparant deux clignements successifs et pour déclencher une alarme renforcée dès que ces intervalles de temps présentent une irrégularité qui dépasse un seuil déterminé.

13. Dispositif selon l'une quelconque des revendications 8 à 12, **caractérisé en ce qu'**il comporte des moyens pour réactualiser en continu les données concernant au moins un des paramètres suivants : déplacements horizontaux, déplacements verticaux, durées des clignements des yeux, intervalles entre clignements successifs, afin de perfectionner les approximations des valeurs normales du paramètre impliqué pour le conducteur effectivement présent et à l'état éveillé.

14. Dispositif selon l'une quelconque des revendications 8 à 13, **caractérisé en ce que** ledit ensemble capteur opto-électronique(10)- unité électronique (19) produit un signal vidéo comportant une succession de trames correspondantes de même nature à succession de lignes constituées par une succession de pixels et traite le dit signal video pour successivement :
- déduire, des variations de la valeur ou intensité de chaque pixel entre une trame et la trame correspondante antérieure,
• d'une part, un signal binaire, noté DP, dont les deux valeurs possibles sont représentatives, l'une, d'une variation significative de la valeur du pixel et, l'autre, d'une non-variation significative de cette valeur, et
• d'autre part, un signal numérique, noté CO, à nombre réduit de valeurs possibles, ce signal étant représentatif de la grandeur de cette variation de la valeur du pixel ;
- répartir suivant une matrice, par roulement, des valeurs de ces deux signaux DP et CO pour une même trame qui défile à travers la matrice ; et
- déduire, de cette répartition matricielle, le déplacement recherché et ses paramètres de localisation et de direction.

15. Dispositif selon l'une quelconque des revendications 8 à 14, **caractérisé en ce que** ledit capteur (10), ladite électronique associée (19) et ledit circuit intégré sont constituées par une puce électronique (chip) disposée à l'intérieur du boitier du rétroviseur (8).

16. Rétroviseur de véhicule automobile, **caractérisé en ce que** son miroir est constitué par une glace sans tain (9) et **en ce qu'**il comporte, derrière cette glace, un capteur optoélectronique (10) qui coopère avec une unité électronique (19), produit un signal vidéo comportant une succession de trames correspondantes de même nature à succession de lignes constitué par une succession de pixels et traite le dit signal video pour successivement :
- déduire, des variations de la valeur ou intensité de chaque pixel entre une trame et la trame correspondante antérieure,
• d'une part, un signal binaire, noté DP, dont les deux valeurs possibles sont représentatives, l'une, d'une variation significative de la valeur du pixel et, l'autre, d'une non-variation significative de cette valeur,et
• d'autre part, un signal numérique, noté CO, à nombre réduit de valeurs possibles, ce signal étant représentatif de la grandeur de cette variation de la valeur du pixel ;
- répartir suivant une matrice, par roulement, des valeurs de ces deux signaux DP et CO pour une même trame qui défile à travers la matrice ;
- déduire, de cette répartition matricielle, le déplacement recherché et ses paramètres de localisation et de direction ; et
- déclencher un dispositif d'alarme (22) dès que ladite unité détermine que les mouvements verticaux des paupières d'une personne regardant la face avant (9*a*) de ladite glace correspondent à une durée des clignements des yeux qui dépasse un seuil prédéterminé inclus dans l'intervalle temporel compris entre la durée des clignements d'une personne éveillée et celle d'une personne qui somnole..

17. Rétroviseur de véhicule automobile selon la revendication 16, **caractérisé en ce que** ledit capteur (10) ), ladite électronique associée (19) et ledit circuit intégré sont constituées par une puce électronique (chip) disposée à l'intérieur du boitier du rétroviseur (8).

18. Rétroviseur de véhicule automobile selon la revendication 16 ou 17, **caractérisé en ce qu'**il porte en outre au moins une diode (20) électroluminescente au moins dans l'infra-rouge qui est activée au moins lorsque la luminosité ambiante devient insuffisante pour éclairer le visage du conducteur et **en ce que** ledit capteur optoélectronique (10) est sensible entre autres, aux radiations infra-rouges émises par ladite diode.

## Claims

1. A method for continuons monitoring of the driver's watchfulness in an automotive vehicle, in order to detect and to prevent any possible sleepiness of the former, consisting in
- producing a video signal, significant in real time of the successive pictures of at least the driver's face;
- processing this signal, successively and continuously, to
• detect in this signal the portion corresponding effectively to the image of the driver's head,
• determine the value of a parameter in relation to the blinking of the eyelids, with a notable modification when the driver passes from the awake condition to the sleepy condition, on either side of a threshold, and
• locate in real time when the value of this parameter exceeds this significant threshold of the driver's transition from awake condition to sleepy condition; and
- triggering, in response to the threshold being exceeded, an alarm capable of waking up the driver;
and which is **characterised in that**
- on the one hand, the video signal is produced by using an optoelectronic sensor integral with a rear-mirror on the automotive vehicle, dimensioned and arranged to essentially receive the driver's face image when seated and whose optic axis for receiving the light rays is directed towards the' driver's head when the rear-mirror is oriented correctly; and
- on the other, processing the said video signal consists, after detecting the presence of the driver on his/her seat, successively and continuously, in:
• detecting, from an analysis of moving pixels between two successive frames of same nature of said video signal, the horizontal movements of the driver, in order to centre the face of the said driver within the successive corresponding frames of the video signal,
• detecting, from an analysis of moving pixels between two successive frames of same nature of said video signal, the vertical movements in the face, thus centred, of the driver in order to centre his/her eyes,
• determining, from an analysis of moving pixels between two successive frames of same nature of said vîdeo signal, the successive blinking durations of the eyes, thus centred, of the driver, whereby the said durations make up the said parameter,
• comparing these successive blinking durations, thus determined,with a threshold significant of the transition from awake condition to sleepy condition of the driver, and triggering, when the blinking durations exceed the said threshold upwards, an alarm capable of waking up the driver.

2. A method according to claim 1, **characterised in that** the said sensor is placed in the casing of the rear mirror behind the mirror of the former which consists of a two-way mirror, the réception optic axis (2a) of said sensor being symmetrical to an axis (2b) oriented in the vertical median plane of said vehicle, related to an orthogonal axis (6) to said twoway mirror.

3. A method according to claim 1 or 2, **characterised in that** the presence of the driver on his/her seat is detected while determining the number of pixels corresponding in the successive frames of the same nature in the video signal for which a significant movement is detected and by comparing this number with the total number of pixels per frame of the video signal, in order to determine whether the ratio between the number of pixels representing a displacement and the total number of pixels per frame exceeds a threshold significant of the transition from the drive@s absence on his/her seat to the presence of a driver in his/her seat.

4. A method according to claim 1, 2 or 3; **characterised in that**, between the détection phases of horizontal displacements in order to centre the driver's face, and the détection of vertical displacements, in order to frame the drivers eyes, a wide centring phase of the eyes has been provided while being limited to a portion of the centred face, covering the eyes and their immédiate surrounding, by applying the anthropometric ratio between the said portion and the entire face of a person.

5. A method according to any one of the previous claims, **characterised in that** simultaneously to the détermination phase of the blinking durations, a détermination phase of time intervals between two successive winks has been provided and a reinforced alarm should be triggered as soon as those time intervals present an irregularity which exceeds a set threshold.

6. A method according to any one of the previous claims, **characterised in that** continuons update of the data is performed, regarding at least one of the following parameters: horizontal displacements, vertical displacements, blinking durations, intervals between successive winks, in order to perfect the approximations of the normal values of these parameters for the driver actually present and in awake condition.

7. A method according to any one of the previous claims **characterised in that** the different successive phases of the process are performed using successive computer programmes which would process successive values of the corresponding pixels of the frames of same nature in the video signal obtained from the said sensor.

8. A device for continuons monitoring of the drive@s watchfulness in an automotive vehicie, in order to detect and to prevent any possible sleepiness of the former, implementing the method according to any one of claims 1 to 7 and which is **characterised in that** it comprises, in combination:
a) an optoelectronic sensor (10) which, in combination with associated electronic circuitry (19), prepares, in response to the receptîon of light rays, a vi.deo signal with frames of the same nature, or corresponding, successive frames, whereas the said sensor is integral with a vehicle rear mirror (8) and dimensioned and arranged to essentially receive the drive@s face image when seated and whose optic axis (1 Ob) for receiving the light rays is directed towards the driver's head (T) when the rear mirror is suitably oriented; and
b) at least an integrated circuit including
- means to detect the driver's presence on his/her seat in the vehicle, and to prepare a presence signal;
- means, actuated by this presence signal, to detect on the basis of an analysis of moving pixels between two successive frames of same nature of said video signal, the horizontal displacements of the said driver, in order to centre his/her face (V) in the successive frames of same nature in the said video signal and to prepare a signal at the end of the face centring;
- means, actuated by the said end signal of the face centring, to detect on the basis of an analysis of movihg pixels between two successive frames of same nature of the portion of the successive frames of same nature in the said video signal corresponding to the face centring, the vertical displacements in the face, thus centred, of the driver, in order to centre the eyes (U) of the said driver, within the said portion of the frames of this signal and to prepare a centring end signal of the driver's eyes;
- means, actuated by the. said eyes centring end signal, to determine on the basis of an analysis of moving pixels between two successive frames of same nature of the portion of the successive frames of same nature in the said video signal corresponding to the eyes centring, the successive durations of the driver's blinking;
- means to compare these successive blinking durations, thus determined, with a threshold significant of the transition from awake condition to sleepy condition of the driver; and
- means to trigger, when the blinking durations exceed the saîd threshold, an alarm (22) capable of waking up the driver.

9. A device according to claim 8, **characterised in that** the said sensor (10) is placed in the casing of the rear mirror (8) behind the mirror of the former which consists of a two-way mirror (9), said sensor (10) being carried by a first extremity of a first rod (13) crossing, through a trunnion (17), a clamp carried by the rear mirror casing (8), inside of it, the second extremity of this rod (13) being freely hinged, with a seam (14a, 14b), to the first extremity of a second rod (12) crossing, through a trunnion (15), the rear mirror casing (8), while, the second extremity of said second rod (12) is fixed to the bodywork of the vehicle (in 5) above the front windscreen, in order that the réception optic axis (2a) of said sensor being symmetrical to an axis (2b) oriented in the vertical median'plane of said vehicle, related to an orthogonal axis (6) to said two-way mirror.

10. A device according to claim 8 or 9, **characterised in that** the said means to detect the presence of the driver on his/her seat and to prepare a presence signal consist of means to determine the number of pixels in the successive frames of the same nature in the said video signal for which a significant movement is detected, means to compare this number with the total number of pixels per frame in the video signal, in order to determine whether the ratio between the number of pixels representing a displacement and the total number of pixels per frame exceeds a threshold significant of the transition from the driver's absence on his/her seat to the presence of a driver in his/her seat.

11. A device according to claim 8, 9 or 10, **characterised in that** it comprises moreover, means, actuated by the said face centring end signal, in order to select, in the said portion of the excessive frames of the said video signal corresponding to the face centring, a reduced portion corresponding to a wide or rough centring, of the driver's eyes covering the eyes and their immédiate surrounding by applying the anthropometric ratio between the said wide centring and the entire face of a person and means to prepare a wide centring end signal of the eyes, whereas this signal actuates the said means to detect the vertical displacements in the driver's face.

12. A device according to any one of claims 8 to 11, **characterised in that** it comprises means, operating in parallel with the said means to determine the successive blinking durations and hence actuated by the said eyes centring end signal, in order to determine the time intervals between two successive winks and to trigger a reinforced alarm as soon as these time intervals show an irregularity exceeding a set threshold.

13. A device according to any one of claims 8 to 12, **characterised in that** it comprises means for continuons update of the data regarding at least one of the following parameters: horizontal displacementà, vertical displacements, blinking durations, intervals between successive winks, in order to perfect the approximations of the normal values of the parameter involved for the driver actually present and in awake condition.

14. A device according to any one of claims 8 to 13, **characterised in that** the optoelectronic sensor (10) - electronic unit (19) assembly produces a video signal comprising a succession of corresponding frames of same nature with a succession of lines made of a succession of pixels and processes the said video signal in order to successively
- deduct, from the variations in the value or intensité of each pixel between a frame and the corresponding former frame
• on the one hand, a binary signal, noted DP, whose both possible values are significant, one of a significant variation in the pixel value and the other, of a non-significant variation of the pixel value and
• on the other, a digital signal, noted CO, with a reduced number of possible values, whereby this signal is significant of the magnitude of this variation in the pixel value;
- distribute according to a matrix, on a noria basis, values for both these sîgnals DP and CO for the same frame scrolling through the matrix; and
- derive, from this matrix distribution, the requested displacement and its location and direction parameters.

15. A device according to any one of claims 8 to 14, **characterised in that** said sensor (10), said associated electronic circuitry (19) and said integrated circuit are constituted by an electronic chip arranged in the rear mirror casing (8).

16. A rear mirror for automotive vehicie, **characterised in that** its mirror consists of a two-way mirror (9) and **in that** it comprises, behind this two-way mirror, an optoelectronic sensor (10) cooperating with an electronic unit (19) producing a video signal comprising a succession of corresponding frames of same nature with a succession of lines made of a succession of pixels and processing the said video signal in order to successively
- deduct, from the variations in the value or intensité of each pixel between a frame and the corresponding former frame
• on the one hand, a binary signal, noted DP, whose both possible values are significant, one of a significant variation in the pixel value and the other, of a non-significant variation of the pixel value, and
• on the other, a digital signal, noted CO, with a reduced number of possible values, whereby this signal is significant of the magnitude of this variation in the pixel value;
- distribute according to a matrix, on a noria basis, values for both these signals DP and CO for the same frame scrolling through the matrix; and
- derive, from this matrix distribution, the requested displacement and its location and direction parameters: and
- trigger an alarm device (22) as soon as the said unit determines that the vertical movements of a person's eyelids looking at the front face (9a) of the said mirror correspond to a blinking duration exceeding a pre-set threshold included in the time interval comprised between the duration a woken person's blinking and that of a person falling asleep.

17. A rear mirror for automotive vehicle according to claim 16, **characterised in that** said sensor (10), said associated electronic circuitry (19) and said integrated circuit are constituted by an electronic chip arranged in the rear mirror casing (8).

18. A rear mirror for automotive vehicie according to claim 16 or 17, **characterised in that** it carries moreover at least one light-emitting diode (20) at least in,the infrared range which is actuated at least when the surrounding luminosity becomes insufficient to light the driver's face and **in that** the said optoelectronic sensor (1 0) is sensitive, among other things, to the infrared radiations transmitted by the said diode.

## Patentansprüche

1. Verfahren zur ständigen Überwachung des Wachsamkeitszustandes des Fahrers eines Kraftfahrzeugs, um eine mögliche Neigung zum Einschlafen desselben zu erfassen und dieser vorzubeugen,
darin bestehend
- in Echtzeit ein Videosignal zu erzeugen, das für die aufeinanderfolgenden Bilder von mindestens dem Gesicht des Fahrers repräsentativ ist;
- dieses Signal aufeinanderfolgend und kontinuierlich zu bearbeiten, um
• in diesem Signal den Abschnitt zu erfassen, der tatsächlich dem Bild des Kopfes des Fahrers entspricht,
• den Wert eines Parameters für den Lidschlag zu bestimmen, der sich beim Übergang vom wachen in den schläfrigen Zustand des Fahrers beiderseits eines Schwellenwerts stark verändert, und
• in Echtzeit die Überschreitung dieses Schwellenwerts für den Übergang vom wachen in den schläfrigen Zustand des Fahrers durch den Wert dieses Parameters zu erfassen; und
- als Reaktion auf die Überschreitung dieses Schwellenwerts einen Alarm auszulösen, der den Fahrer aufzuwecken in der Lage ist;
**dadurch gekennzeichnet, daß**
- einerseits das Videosignal erzeugt wird, indem ein optoelektronischer Sensor verwendet wird, der mit einem Rückspiegel des Kraftfahrzeugs fest verbunden und derart dimensioniert und angeordnet ist, daß er im wesentlichen das Bild des Gesichtes des Fahrers, der sich auf seinem Sitz befindet, empfängt, und dessen optische Achse für die Aufnahme der Lichtstrahlen zu dem Kopf des Fahrers gerichtet ist, wenn der Rückspiegel richtig ausgerichtet ist; und
- andererseits die Bearbeitung des Videosignals, nachdem die Anwesenheit des Fahrers an seinem Platz erfasst wurde, aufeinanderfolgend und kontinuierlich darin besteht,
• aus einer Analyse der Pixel bei der Bewegung zwischen zwei aufeinanderfolgenden Rastern derselben Art des Videosignals die horizontalen Bewegungen des Fahrers zu erfassen, um das Gesicht desselben in den entsprechenden aufeinanderfolgenden Rastern des Videosignals einzugrenzen,
• aus einer Analyse der Pixel bei der Bewegung zwischen zwei aufeinanderfolgenden Rastern derselben Art des Videosignals die vertikalen Bewegungen in dem auf diese Weise eingegrenzten Gesicht des Fahrers zu erfassen, um die Augen desselben einzugrenzen,
• aus einer Analyse der Pixel bei der Bewegung zwischen zwei aufeinanderfolgenden Rastern derselben Art des Videosignals die aufeinanderfolgenden Zeitdauern des Zwinkerns der auf diese Weise eingegrenzten Augen desselben zu bestimmen, wobei diese Zeitdauern den erwähnten Parameter darstellen,
• diese aufeinanderfolgenden Zeitdauern des auf diese Weise bestimmten Zwinkerns mit einem Schwellenwert zu vergleichen. der für den Übergang vom wachen in den schläfrigen Zustand des Fahrers repräsentativ ist, und
• einen Alarm, der den Fahrer aufweckt, auszulösen, wenn die Zwinkerdauer den Schwellenwert im oberen Wertebereich überschreitet.

2. Verfähren nach Anspruch 1, **dadurch gekennzeichnet, daß** der Sensor in dem Gehäuse des Rückspiegels hinter dem Glas desselben angeordnet ist, welches von einem Spiegel ohne Belag gebildet wird, wobei die optische Empfangeachse (2a) des Sensors zu einer Achse (2b) symmetrisch ist, die in der mittleren Vertikalebene des Fahrzeugs in bezug auf eine zu dem belaglosen Spiegel orthogonale Achse (6) ausgerichtet ist.

3. Verfahren nach Anspruch 1 oder 2, **dadurch gekennzeichnet, daß** die Anwesenheit des Fahrers an seinem Platz erfasst wird, indem die Anzahl von entsprechenden Pixeln in den aufeinanderfolgenden Rastern derselben Art des Videosignals bestimmt wird, für die eine signifikante Bewegung erfasst wird, und indem diese Anzahl mit der Gesamtpixelzahl pro Raster des Videosignals verglichen wird, um zu bestimmen, ob das Verhältnis zwischen der Anzahl von Pixeln, die eine Verschiebung darstellen, und der Gesamtpixelzahl pro Raster eine Schwelle überschreitet, die für den Übergang der Abwesenheit eines Fahrers an seinem Platz zur Anwesenheit eines Fahrers an seinem Platz repräsentativ ist.

4. Verfahren nach Anspruch 1, 2 oder 3, **dadurch gekennzeichnet, daß** zwischen den Phasen der Erfassung der horizontalen Bewegungen, um das Gesicht des Fahrers einzugrenzen, und der Erfassung der vertikalen Bewegungen, um die Augen desselben einzugrenzen, eine Phase der breiten Eingrenzung der Augen vorgesehen ist, indem ein Abschnitt des eingegrenzten Gesichtes herangezogen wird, der die Augen und ihre unmittelbare Umgebung umfasst, indem das anthropometrische Verhältnis zwischen dem Abschnitt und dem ganzen Gesicht einer Person angewandt wird.

5. Verfahren nach einem der vorstehenden Ansprüche, **dadurch gekennzeichnet, daß** gleichzeitig mit der Phase der Bestimmung der Zeitdauern des Augenzwinkerns eine Phase zur Bestimmung der Zeitintervalle zwischen zwei aufeinanderfolgenden Zwinkervorgängen derselben vorgesehen ist, und ein stärkerer Alarm ausgelöst wird, sobald diese Zeitintervalle eine Unregelmäßigkeit aufweisen, die einen bestimmten Schwellenwert überschreitet.

6. Verfahren nach einem der vorstehenden Ansprüche, **dadurch gekennzeichnet, daß** ständig jene Daten wieder aktualisiert werden, die zumindest einen der folgenden Parameter betreffen: horizontale Bewegungen, vertikale Bewegungen, Zeitdauer des Augenzwinkerns, Intervalle zwischen aufeinanderfolgenden Zwinkervorgängen, um die Annäherungen der Normalwerte dieser Parameter für den tatsächlich anwesenden und sich im Wachzustand befindlichen Fahrer zu verbessern.

7. Verfahren nach einem der vorstehenden Ansprüche, **dadurch gekennzeichnet, daß** die verschiedenen aufeinanderfolgenden Phasen des Verfahrens mit Hilfe von aufeinanderfolgenden Informatikprogrammen durchgeführt werden, die sich auf die Bearbeitung der aufeinanderfolgenden Werte der entsprechenden Pixel der Raster derselben Art des Videosignals beziehen, welches von dem Sensor erhalten wird.

8. Vorrichtung zur kontinuierlichen Überwachung des Wachsamkeitszustandes des Fahrers eines Kraftfahrzeugs, um eine eventuelle Neigung zum Einschlafen desselben zu erfassen und dieser vorzubeugen, welche das Verfahren nach einem der Ansprüche 1 bis 7 anwendet und die **dadurch gekennzeichnet ist, daß** sie in Kombination umfasst:
a) einen optoelektronischen Sensor (10), der in Kombination mit einer angeschlossenen Elektronik (19) als Reaktion auf den Erhalt von Lichtstrahlen ein Videosignal mit Rastern derselben Art oder entsprechenden, aufeinanderfolgenden ausarbeitet, wobei der Sensor mit einem Rückspiegel (8) des Kraftfahrzeugs verbunden und derart dimensioniert und angeordnet ist, um im wesentlichen das Bild des Gesichts des Fahrers, der sich auf seinem Sitz befindet, zu empfangen, und dessen optische Achse (10b) zur Aufnahme der Lichtstrahlen zu dem Kopf (T) des Fahrers gerichtet ist, wenn der Rückspiegel richtig ausgerichtet ist; und
b) mindestens einen integrierten Schaltkreis, umfassend:
- Mittel zur Erfassung der Anwesenheit des Fahrers an seinem Platz in dem Fahrzeug und zur Ausarbeitung eines Anwesenheitssignals;
- Mittel, die durch dieses Anwesenheitssignal aktiviert werden, um aus einer Analyse der Pixel bei der Bewegung zwischen zwei aufeinanderfolgenden Rastern derselben Art des Videosignals die Horizontalbewegungen des Fahrers zu erfassen, um das Gesicht (v) desselben in den aufeinanderfolgenden Rastern derselben Art des Videosignals einzugrenzen und ein Endsignal der Gesichtseingrenzung auszuarbeiten;
- Mittel, die durch das Endsignal der Gesichtseingrenzung aktiviert werden, um aus einer Analyse der Pixel bei der Verschiebung zwischen zwei aufeinanderfolgenden Rastern derselben Art des Abschnittes der aufeinanderfolgenden Raster derselben Art des der Gesichtseingrenzung entsprechenden Videosignals die Vertikalbewegungen in dem auf diese Weise eingegrenzten Gesicht des Fahrers zu erfassen, um die Augen (U) desselben in dem Abschnitt der Raster dieses Signals einzugrenzen und ein Endsignal der Eingrenzung der Augen des Fahrers auszuarbeiten;
- Mittel, die durch das Endsignal der Augeneingrenzung aktiviert werden, um aus einer Analyse der Pixel bei der Bewegung zwischen zwei aufeinanderfolgenden Rastern derselben Art des Abschnittes der aufeinanderfolgenden Raster derselben Art des Videosignals, das der Eingrenzung der Augen entspricht, die aufeinanderfolgenden Zeitdauern der Zwinkervorgänge der Augen des Fahrers zu bestimmen;
- Mittel, um diese aufeinanderfolgenden Zwinkerzeitdauern, die auf diese Weise bestimmt wurden, mit einem Schwellenwert zu vergleichen, der für den Übergang vom wachen in den schläfrigen Zustand des Fahrers repräsentativ ist; und
- Mittel, um, wenn die Zwinkerzeitdauern den Schwellenwert überschreiten, einen Alarm (22) auszulösen, der den Fahrer aufweckt.

9. Vorrichtung nach Anspruch 8, **dadurch gekennzeichnet, daß** der Sensor (10) in dem Gehäuse des Rückspiegels (8) hinter dem Spiegel desselben angeordnet ist, welcher ein Spiegel (9) ohne Belag ist, wobei dieser Sensor (10) von einem ersten Ende einer ersten Stange (13) getragen wird, die über ein Kugelgelenk (17) einen Bügel (16) durchquert, der von dem Gehäuse des Rückspiegels (8) im Inneren desselben getragen wird, wobei das zweite Ende dieser Stange (13) mit Hilfe eines Gelenks (14a, 14b) am ersten Ende einer zweiten Stange (12) frei angelenkt ist, die über ein Kugelgelenk (15) das Gehäuse des Rückspiegels (8) durchquert, während das zweite Ende der zweiten Stange (12) an der Karosserie des Fahrzeugs (bei 5) über der Windschutzscheibe befestigt ist, so daß die optische Aufnahmeachse (2a) des Sensors zu einer Achse (2b) symmetrisch ist, die in der mittleren Vertikalebene des Fahrzeugs in bezug auf eine orthogonale Achse (6) des belaglosen Spiegels ausgerichtet ist.

10. Vorrichtung nach Anspruch 8 oder 9, **dadurch gekennzeichnet, daß** die Mittel zur Erfassung der Anwesenheit des Fahrers an seinem Platz und zur Ausarbeitung eines Anwesenheitssignals von Mitteln zur Bestimmung der Anzahl von Pixeln in den aufeinanderfolgenden Rastern derselben Art des Videosignals, für die eine signifikante Bewegung erfasst wird, von Mitteln zum Vergleichen dieser Anzahl mit der Gesamtpixelzahl des Videosignals pro Raster gebildet wird, um zu bestimmen, ob das Verhältnis zwischen der Pixelanzahl, die einer Verschiebung entspricht, und der Gesamtpixelzahl pro Raster einen Schwellenwert überschreitet, der für den Übergang vom Zustand der Abwesenheit eines Fahrers an seinem Platz in den Zustand der Anwesenheit eines Fahrers an seinem Platz repräsentativ ist.

11. Vorrichtung nach Anspruch 8, 9 oder 10, **dadurch gekennzeichnet, daß** sie ferner Mittel umfasst, die durch das Endsignal der Gesichtseingrenzung aktiviert werden, um in dem Abschnitt der aufeinanderfolgenden Raster des Videosignals, das der Eingrenzung des Gesichts entspricht, einen kleineren Abschnitt auszuwählen, der einer breiten oder groben Eingrenzung der Augen des Fahrers entspricht und die Augen und ihre unmittelbare Umgebung durch Anlegen des anthropometrischen Verhältnisses zwischen der breiten Eingrenzung und dem ganzen Gesicht einer Person umfasst, und Mittel, um ein Endsignal der breiten Eingrenzung der Augen auszuarbeiten, wobei dieses Signal die Mittel zur Erfassung der Vertikalbewegungen im Gesicht des Fahrers aktiviert.

12. Vorrichtung nach einem der Ansprüche 8 bis 11, **dadurch gekennzeichnet, daß** sie Mittel umfasst, die parallel mit den Mitteln zur Bestimmung der aufeinanderfolgenden Zeitdauern der Augenzwinkervorgänge funktionieren und somit von dem Endsignal der Augeneingrenzung aktiviert werden, um die Zeitintervalle zwischen zwei aufeinanderfolgenden Zwinkervorgängen zu bestimmen und einen stärkeren Alarm auszulösen, sobald diese Zeitintervalle eine Unregelmäßigkeit aufweisen, die über einen bestimmten Schwellenwert hinausgeht.

13. Vorrichtung nach einem der Ansprüche 8 bis 12, **dadurch gekennzeichnet, daß** sie Mittel zur kontinuierlichen Wiederaktualisierung der Daten umfasst, die mindestens einen der folgenden Parameter betreffen: Horizontalbewegungen, Vertikalbewegungen, Dauer des Augenzwinkerns, Intervalle zwischen aufeinanderfolgenden Zwinkervorgängen, um die Annäherungen der Normalwerte des Parameters zu verbessern, der für den tatsächlich anwesenden und sich im Wachzustand befindlichen Lenker gilt.

14. Vorrichtung nach einem der Ansprüche 8 bis 13, **dadurch gekennzeichnet, daß** die Einheit optoelektronischer Sensor (10) - Elektronikeinheit (19) ein Videosignal erzeugt, das eine Aufeinanderfolge von entsprechenden Rastern derselben Art mit einer Aufeinanderfolge von Zeilen, die von einer Aufeinanderfolge von Pixeln gebildet werden, umfasst, und das Videosignal bearbeitet, um aufeinanderfolgend:
- aus den Veränderungen des Wertes oder der Stärke jedes Pixels zwischen einem Raster und dem entsprechenden vorhergehenden Raster,
• einerseits ein Binärsignal, mit DP bezeichnet, dessen beide möglichen werte einer für eine signifikante Veränderung des Wertes des Pixels und der andere für eine signifikante Nichtveränderung dieses Wertes repräsentativ sind, und
• andererseits ein Digitalsignal, mit CO bezeichnet, das eine'verringerte Anzahl möglicher Werte aufweist, wobei dieses Signal für die Größe dieser Veränderung des Wertes des Pixels repräsentativ ist, abzuleiten,
- gemäß einer Matrix durch Rollen Werte dieser beiden Signale DP und CO für ein und denselben Raster, der über die Matrix abläuft, zu verteilen; und
- von dieser Matrixverteilung die gewünschte Verschiebung und ihre Lokalisations- und Ausrichtungsparameter abzuleiten.

15. Vorrichtung nach einein der Ansprüche 8 bis 14, **dadurch gekennzeichnet, daß** der Sensor (10), die zugehörige Elektronik (19) und der integrierte Schaltkreis aus einem Elektronikchip gebildet sind, der im Inneren des Gehäuses des Rückspiegels (8) angeordnet ist.

16. Rückspiegel eines Kraftfahrzeugs, **dadurch gekennzeichnet, daß** sein Spiegel von einem belaglosen Glas (9) gebildet ist und daß er hinter diesem Glas einen optoelektronischen Sensor (10) umfasst, der mit einer Elektronikeinheit (19) zusammenwirkt, ein Videosignal erzeugt, das eine Aufeinanderfolge von entsprechenden Rastern derselben Art mit einer Aufeinanderfolge von Zeilen, gebildet aus einer Aufeinanderfolge von Pixeln, umfasst, und das Videosignal bearbeitet, um aufeinanderfolgend.
- aus den Veränderungen des Wertes oder der Stärke jedes Pixels zwischen einem Raster und dem entsprechenden vorhergehenden Raster,
• einerseits ein Binärsignal, mit DP bezeichnet, dessen beide möglichen Werte einer für eine signifikante Veränderung des Wertes des Pixels und der andere für eine signifikante Nichtveränderung dieses Wertes repräsentativ sind, und
• andererseits ein, Digitalsignal, mit CO bezeichnet, das eine verringerte Anzahl möglicher Werte aufweist, wobei dieses Signal für die Größe dieser Veränderung des Wertes des Pixels repräsentativ ist, abzuleiten,
- gemäß einer Matrix durch Rollen Werte dieser beiden Signale DP und CO für ein und denselben Raster, der über die Matrix abläuft, zu verteilen; und
- von dieser Matrixverteilung die gewünschte Verschiebung und ihre Lokalisations- und Ausrichtungsparameter abzuleiten; und
- eine Alarmvorrichtung (22) ausaulösen, sobald die Einheit feststellt, daß die Vertikalbewegungen der Augenlider einer Person, die die Vorderfläche (9a) des Glases betrachtet, einer Dauer der Zwinkervorgänge der Augen entsprechen, die einen vorbestimmten, in dem von der Dauer der Zwinkervorgänge einer wachen Person und jener einen Person, die schläfrig ist, begrenzten Zeitintervall liegenden Schwellenwert überschreitet.

17. Rückspiegel eines Kraftfahrzeugs nach Anspruch 16, **dadurch gekennzeichnet, daß** der Sensor (10), die zugehörige Elektronik (19) und die integrierte Schaltung aus einem Elektronikchip gebildet sind, der im Inneren des Gehäuses des Rückspiegels (8) angeordnet ist.

18. Rückspiegel eines Kraftfahrzeugs nach Anspruch 16 oder 17, **dadurch gekennzeichnet, daß** er ferner mindestens eine Leuchtdiode (20) zumindest im Infrarotbereich trägt, die zumindest dann aktiviert wird, wenn die Helligkeit der Umgebung unzureichend wird, um das Gesicht des Fahrers zu erhellen, und daß der optoelektronische Sensor (10) unter anderem für Infrarotstrahlen empfindlich ist, die von der Diode ausgesandt werden.
